# EUROPEAN PATENT APPLICATION

(11) **EP 4 749 645 A1**
(43) Date of publication of application: **27.05.2026**
(21) Application number: 25217412.3
(22) Date of filing: 20.11.2025
(51) Int. Cl.: G16H 20/40, G16H 40/63

(54) **PROBLEM-SOLVING LEVEL BASED ON THE BALANCE OF UNKNOWNS AND DATA STREAMS**

(30) Priority: 20.11.2024 US 202418954197
(71) Applicant: Cilag GmbH International, 6300 Zug (CH)
(72) Inventor: SHELTON, IV, Frederick E., Cincinnati, 45242 (US); JONES, Shannon L., Cincinnati, 45242 (US); BRADY, John E., Cincinnati, 45242 (US)
(74) Representative: Carpmaels & Ransford LLP

(57) **Abstract**

Systems, methods, and/or instrumentalities disclosed herein may determine a problem-solving level based on the balance of unknowns and data streams. The device may receive a first dataflow from a first surgical element. The device may determine that a physiological parameter of the patient exceeds a patient safety threshold. The device may determine a second dataflow associated with a second surgical element. The determination may be based on an indication of a relational link. The device may transmit a configuration message including an indication that the physiological parameter of the patient exceeded the patient safety threshold. The device may transmit, to the second surgical element, a control message including an indication of the control data associated with the first surgical element. The device may cause the output characteristic associated with the second surgical element to be adjusted based on the control data associated with the first surgical element.

## Description

### CROSS-REFERENCE TO RELATED APPLICATIONS

This application claims the benefit of the following, the disclosures of which are incorporated herein by reference in its entirety:
- Provisional U.S. Patent Application No. 63/602,040, filed November 22, 2023,
- Provisional U.S. Patent Application No. 63/602,028, filed November 22, 2023,
- Provisional U.S. Patent Application No. 63/601,998, filed November 22, 2023,
- Provisional U.S. Patent Application No. 63/602,003, filed November 22, 2023,
- Provisional U.S. Patent Application No. 63/602,006, filed November 22, 2023,
- Provisional U.S. Patent Application No. 63/602,011, filed November 22, 2023,
- Provisional U.S. Patent Application No. 63/602,013, filed November 22, 2023,
- Provisional U.S. Patent Application No. 63/602,037, filed November 22, 2023,
- Provisional U.S. Patent Application No. 63/602,007, filed November 22, 2023,
- Provisional U.S. Patent Application No. 63/603,031, filed November 27, 2023, and
- Provisional U.S. Patent Application No. 63/603,033, filed November 27, 2023.

This application is related to the following, filed contemporaneously, the contents of each of which are incorporated by reference herein:
• U.S. Patent Application No. 18/954,186, filed November 20, 2024, entitled METHOD FOR MULTI-SYSTEM INTERACTION, and
• U.S. Patent Application No. 18/954,192, filed November 20, 2024, entitled AUGMENTING DATAFLOWS TO REBALANCE THE NUMBER OF UNKNOWNS AND DATAFLOWS.

### BACKGROUND

Surgical procedures are typically performed in surgical operating theaters or rooms in a healthcare facility such as, for example, a hospital. Various surgical devices and systems are utilized in performance of a surgical procedure. In the digital and information age, medical systems and facilities are often slower to implement systems or procedures utilizing newer and improved technologies due to patient safety and a general desire for maintaining traditional practices.

### SUMMARY

A problem-solving level may be determined based on the balance of unknowns and data streams. Instead of replacing a smart device, pausing and/or cancelling a procedure, or continuing a procedure with a reduced number of smart devices, a device (e.g., a surgical computing system) may use sensor data from a second smart device to generate control data for a first smart device. In the event that a surgical computing system detects that a first smart device is transmitting erroneous sensor data (e.g., an erroneous dataflow), the surgical computing system may determine that a second smart device is configured to provide sensor data similar to and/or the same as the failed first smart device. The surgical computing system may transmit a configuration message to the second smart device, requesting that the second device send the related sensor data to the surgical computing system. In examples, a surgical computing system may determine that a second dataflow (e.g., including control data, sensor data, and/or dataflow configuration information) may be added to a first dataflow to resolve an issue during a procedure.

A device may include a processor. The device may be configured to receive a first dataflow from a first surgical element. The first dataflow may be associated with a physiological parameter of a patient. The device may determine based on the first dataflow, that the physiological parameter of the patient exceeds a patient safety threshold. The device may determine a second dataflow associated with a second surgical element. The determination may be based on an indication of a relational link associated with control data for the second surgical element, the first dataflow, and/or the physiological parameter of the patient. The device may transmit, to the second surgical element, a configuration message comprising an indication that the physiological parameter of the patient exceeded the patient safety threshold, and/or a request to configure the second dataflow to receive control data associated with the first surgical element. The device may generate the control data associated with the first surgical element. The control data may indicate an adjustment to an output characteristic associated with the second surgical element. The device may transmit, to the second surgical element, a control message. The control message may include an indication of the control data associated with the first surgical element. The device may cause the output characteristic associated with the second surgical element to be adjusted based on the control data associated with the first surgical element.

In examples, the physiological parameter of the patient may satisfy a threshold if a heart rate exceeds an operational window of 40-120 beats per minute, an oxygen saturation of the patient decreases below 90%, or a core body temperature of the patient is less than 89 degrees Fahrenheit. The configuration message may further include a surgical element ID, a communication protocol, and/or security or access control credentials. The relational link may be determined based on a lookup table (LUT). The LUT may indicate a relationship between the control data for the second surgical element, the first dataflow, and/or the physiological parameter of the patient. The relational link may be determined based on a machine learning (ML) model. The ML model may be trained based on a training data set. The training data set may include a plurality of dataflows and/or a plurality of control data associated with the physiological parameter of the patient.

The device may, based on the first dataflow, determine that the physiological parameter of the patient satisfies a second threshold. The second threshold may be associated with an acceptable operational range. The device may transmit a second configuration message. The second message may include an indication to reconfigure the second dataflow of the second surgical element, to remove the control data associated with the first surgical element. The device may, in response to the determination that the physiological parameter of the patient exceeded the patient safety threshold, transmit an interrogation message to the second surgical element. The interrogation message may request dataflow configuration information for the second surgical element and/or an indication of control data for the second surgical element that is associated with the physiological parameter of the patient. The device may receive an interrogation response indicating at least one physiological parameter of the patient associated with the second surgical element. The dataflow configuration information may include at least one of a communication protocol, a frequency, a destination, or access control credentials.

A device may determine based on a first dataflow, that a physiological parameter of a patient exceeds a patient safety threshold. The device may determine a second dataflow associated with a second surgical element. The determination may be based on an indication of a relational link associated a first surgical element and/or the second surgical element. The device may generate control data associated with the first surgical element. The control data may indicate an adjustment to an output characteristic associated with the second surgical element. The device may transmit a control message to the second surgical element. The control message may include an indication of the control data associated with the first surgical element. The device may cause the output characteristic associated with the second surgical element to be adjusted based on the control data associated with the first surgical element.

### BRIEF DESCRIPTION OF THE DRAWINGS

FIG. 1 is a block diagram of a computer-implemented surgical system.
FIG. 2 shows an example surgical system in a surgical operating room.
FIG. 3 illustrates an example surgical hub paired with various systems.
FIG. 4 shows an example situationally aware surgical system.
FIG. 5 shows an example surgical instrument.
FIGS. 6A-6B illustrate an example operational environments including example data paths for one or more dataflows.
FIG. 7 illustrates an example routine for re-balancing the number of unknowns and dataflows.
FIG. 8 illustrates an example communication diagram for re-balancing the number of unknowns and dataflows.
FIGS. 9A-9B illustrate example aspects of an operational environment for re-balancing a temperature measurement and temperature control.

### DETAILED DESCRIPTION

Operating rooms are becoming more sophisticated with the introduction of smart devices (e.g., interchangeably referred to herein as "surgical elements"). Smart devices may be used and/or adjusted by a health care personnel (HCP), and/or by a surgical computing system during a procedure. Smart devices may include one or more advanced capabilities to significantly enhance the precision, safety, and efficiency of a procedure (e.g., a surgical procedure, diagnostic procedure, therapeutic procedure, preventative procedure and/or the like), while reducing the risk of complications to patients. Examples of smart devices may include robotic surgical systems, navigation systems, smart imaging systems, endoscopic and/or laparoscopic systems, ultrasound scalpels, anesthesia machines, patient monitoring systems (pulse oximeters, blood pressure monitors, EKG monitors, EEG monitors, and/or the like), energy devices (e.g., electrosurgical units, laser surgery systems, and/or the like), infusion pumps, and/or the like.

Smart devices may include one or more sensors. Sensors may be used by smart devices to measure any number of environmental conditions associated with an operational environment. For example, sensors may measure one or more physiological parameters of a patient (e.g., oxygen saturation, blood pressure, respiratory rate, blood sugar, heart rate, a core body temperature, a hydration state and/or the like), an environmental characteristic of the operating room (e.g., a room temperature, a number of HCPs during a procedure, the position of HCPs, a humidity level, air quality, lighting levels, CO2 levels and/or the like) and/or one or more characteristics of a surgical element (e.g., a current, voltage, an pressure, a force applied, a distance, a vibration, an orientation, a flow rate, a status, and/or the like). As an illustrative example an HVAC system may include a temperature, humidity, and/or an air quality sensor. In examples, a robotic surgical system may include a force and/or pressure sensor, a depth sensor, a temperature sensor, a blood flow and/or oxygen sensor, a pH sensor (e.g., to measure blood gas), an electrocardiogram (ECG) sensor, a position sensor (e.g., to measure the position of a robotic arm), and/or the like.

Several smart devices including multiple sensors may be present during a procedure. In many cases, a first smart device and a second smart device may include similar sensors (e.g., each including a temperature sensor, a current sensor, and/or any other sensor) during a procedure. As an illustrative example, during open heart surgery, an operating room may include a ventilator and a pulse oximeter (e.g., among other smart devices). The ventilator may include an air flow sensor to monitor the flow rate of air entering and leaving a patient's lungs, and an oxygen sensor to measure the concentration of oxygen in the air mixture delivered to the patient. The pulse oximeter may measure the oxygen saturation level (SpO2) in a patient's blood.

Occasionally, smart devices and/or their associated sensors may transmit erroneous data. Erroneous data may be caused by, for example, an incorrectly calibrated sensor, a misplaced and/or improperly installed sensor (e.g., interference by a heat source), calibration drift, contamination, electromagnetic interference, a failed sensor, operator error, misalignment (e.g., in the case of a position sensor), and/or the like.

Instead of replacing a smart device, pausing and/or cancelling a procedure, or continuing a procedure with a reduced number of smart devices, a surgical computing system may use sensor data from a second smart device to generate control data for the first smart device. In the event that a surgical computing system detects that a first smart device is transmitting erroneous sensor data (e.g., an erroneous dataflow), the surgical computing system may determine that a second smart device is configured to provide sensor data similar to and/or the same as the failed first smart device. The surgical computing system may transmit a configuration message to the second smart device, requesting that the second device send the related sensor data to the surgical computing system. In examples, a surgical computing system may determine that a second dataflow (e.g., including control data, sensor data, and/or dataflow configuration information) may be added to a first dataflow to resolve an issue during a procedure.

The surgical computing system may determine that a second smart device may provide supplemental and/or an additional sensor data based on one or more determined relationships between smart devices, the surgical computing system, a patient, and/or an HCP. A relationship may be defined in a lookup table (LUT), determined by an HCP, and/or determined by a machine learning (ML) model. A relationship may be determined based on historical data. Historical data may include gathered and/or determined data based on an operational environment including control data, operational data, sensor data, system generated data, training data and/or the like.

To establish a second dataflow from a second smart device, the surgical computing system may transmit an interrogation message and/or a configuration message to the second smart device and/or to another surgical computing system. An interrogation message may, among other things, request confirmation that a smart device includes a compatible and/or available dataflow as indicated by a LUT, determined by an HCP, and/or determined by an ML model. A configuration message may include information associated with configuring a smart device (e.g., reconfiguring a smart device to transmit a dataflow to the surgical computing system). The configuration message may be sent based on one or more determined relationships as described herein. A surgical computing device may receive an interrogation response and/or a configuration response.

Based on the received response, a surgical computing system may receive a second dataflow from a configured smart device (e.g., a second smart device). The second dataflow may be used by the surgical computing system to generate control data for the first smart device (e.g., to compensate for the erroneous sensor data of a first smart device and/or to be used with the first dataflow to solve an issue (e.g., referred to herein as an "unknown"). The control data may be sent to the first smart device. The control data may cause the first smart device to adjust an output characteristic to allow a procedure to continue without interruption.

A more detailed understanding may be had from the following description, given by way of example in conjunction with the accompanying drawings.

### Example Aspects of Surgical Systems

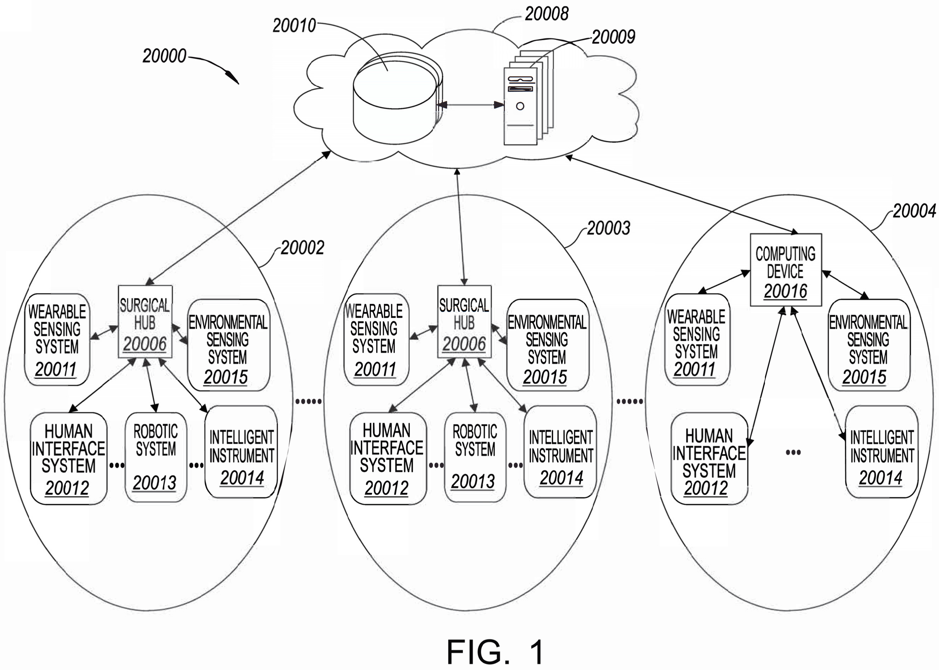

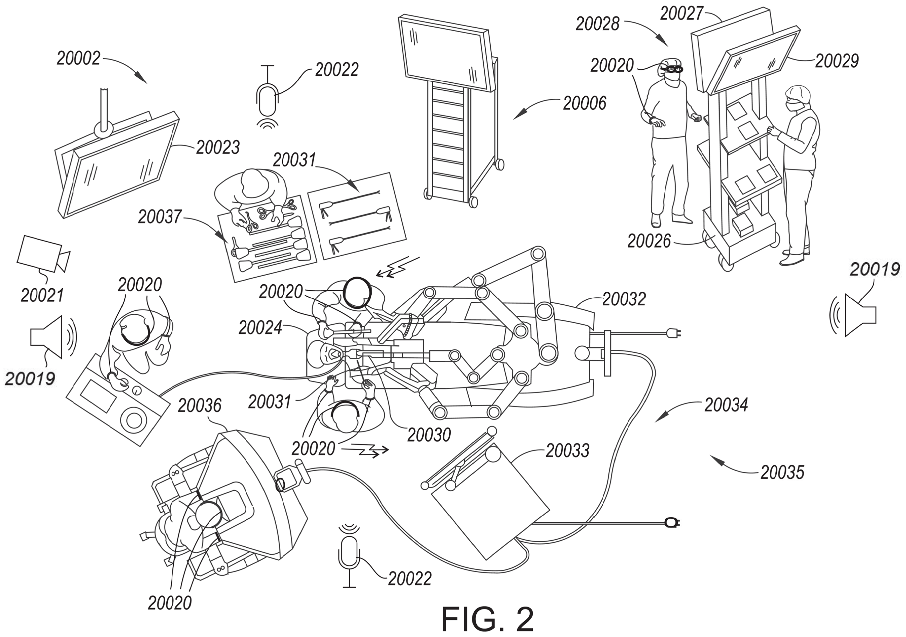

FIG. 1 shows an example computer-implemented surgical system 20000. The example surgical system 20000 may include one or more surgical systems (e.g., surgical sub-systems) 20002, 20003 and 20004. For example, surgical system 20002 may include a computer-implemented interactive surgical system. For example, surgical system 20002 may include a surgical hub 20006 and/or a computing device 20016 in communication with a cloud computing system 20008, for example, as described in FIG. 2. The cloud computing system 20008 may include at least one remote cloud server 20009 and at least one remote cloud storage unit 20010. Example surgical systems 20002, 20003, or 20004 may include one or more wearable sensing systems 20011, one or more environmental sensing systems 20015, one or more robotic systems 20013, one or more intelligent instruments 20014, one or more human interface systems 20012, etc. The human interface system is also referred herein as the human interface device. The wearable sensing system 20011 may include one or more HCP sensing systems, and/or one or more patient sensing systems. The environmental sensing system 20015 may include one or more devices, for example, used for measuring one or more environmental attributes, for example, as further described in FIG. 2. The robotic system 20013 may include a plurality of devices used for performing a surgical procedure, for example, as further described in FIG. 2.

The surgical system 20002 may be in communication with a remote server 20009 that may be part of a cloud computing system 20008. In an example, the surgical system 20002 may be in communication with a remote server 20009 via an internet service provider's cable/FIOS networking node. In an example, a patient sensing system may be in direct communication with a remote server 20009. The surgical system 20002 (and/or various sub-systems, smart surgical instruments, robots, sensing systems, and other computerized devices described herein) may collect data in real-time and transfer the data to cloud computers for data processing and manipulation. It will be appreciated that cloud computing may rely on sharing computing resources rather than having local servers or personal devices to handle software applications.

The surgical system 20002 and/or a component therein may communicate with the remote servers 20009 via a cellular transmission/reception point (TRP) or a base station using one or more of the following cellular protocols: GSM/GPRS/EDGE (2G), UMTS/HSPA (3G), long term evolution (LTE) or 4G, LTE-Advanced (LTE-A), new radio (NR) or 5G, and/or other wired or wireless communication protocols. Various examples of cloud-based analytics that are performed by the cloud computing system 20008, and are suitable for use with the present disclosure, are described in U.S. Patent Application Publication No. US 2019-0206569 A1 (U.S. Patent Application No. 16/209,403), titled METHOD OF CLOUD BASED DATA ANALYTICS FOR USE WITH THE HUB, filed December 4, 2018, the disclosure of which is herein incorporated by reference in its entirety.

The surgical hub 20006 may have cooperative interactions with one of more means of displaying the image from the laparoscopic scope and information from one or more other smart devices and one or more sensing systems 20011. The surgical hub 20006 may interact with one or more sensing systems 20011, one or more smart devices, and multiple displays. The surgical hub 20006 may be configured to gather measurement data from the sensing system(s) and send notifications or control messages to the one or more sensing systems 20011. The surgical hub 20006 may send and/or receive information including notification information to and/or from the human interface system 20012. The human interface system 20012 may include one or more human interface devices (HIDs). The surgical hub 20006 may send and/or receive notification information or control information to audio, display and/or control information to various devices that are in communication with the surgical hub.

For example, the sensing systems may include the wearable sensing system 20011 (which may include one or more HCP sensing systems and/or one or more patient sensing systems) and/or the environmental sensing system 20015 shown in FIG. 1. The sensing system(s) may measure data relating to various biomarkers. The sensing system(s) may measure the biomarkers using one or more sensors, for example, photosensors (e.g., photodiodes, photoresistors), mechanical sensors (e.g., motion sensors), acoustic sensors, electrical sensors, electrochemical sensors, thermoelectric sensors, infrared sensors, etc. The sensor(s) may measure the biomarkers as described herein using one of more of the following sensing technologies: photoplethysmography, electrocardiography, electroencephalography, colorimetry, impedimentary, potentiometry, amperometry, etc.

The biomarkers measured by the sensing systems may include, but are not limited to, sleep, core body temperature, maximal oxygen consumption, physical activity, alcohol consumption, respiration rate, oxygen saturation, blood pressure, blood sugar, heart rate variability, blood potential of hydrogen, hydration state, heart rate, skin conductance, peripheral temperature, tissue perfusion pressure, coughing and sneezing, gastrointestinal motility, gastrointestinal tract imaging, respiratory tract bacteria, edema, mental aspects, sweat, circulating tumor cells, autonomic tone, circadian rhythm, and/or menstrual cycle.

The biomarkers may relate to physiologic systems, which may include, but are not limited to, behavior and psychology, cardiovascular system, renal system, skin system, nervous system, gastrointestinal system, respiratory system, endocrine system, immune system, tumor, musculoskeletal system, and/or reproductive system. Information from the biomarkers may be determined and/or used by the computer-implemented patient and the surgical system 20000, for example. The information from the biomarkers may be determined and/or used by the computer-implemented patient and the surgical system 20000 to improve said systems and/or to improve patient outcomes, for example.

The sensing systems may send data to the surgical hub 20006. The sensing systems may use one or more of the following RF protocols for communicating with the surgical hub 20006: Bluetooth, Bluetooth Low-Energy (BLE), Bluetooth Smart, Zigbee, Z-wave, IPv6 Low-power wireless Personal Area Network (6LoWPAN), Wi-Fi.

The sensing systems, biomarkers, and physiological systems are described in more detail in U.S. App No. 17/156,287 (attorney docket number END9290USNP1), titled METHOD OF ADJUSTING A SURGICAL PARAMETER BASED ON BIOMARKER MEASUREMENTS, filed January 22, 2021, the disclosure of which is herein incorporated by reference in its entirety.

The sensing systems described herein may be employed to assess physiological conditions of a surgeon operating on a patient or a patient being prepared for a surgical procedure or a patient recovering after a surgical procedure. The cloud-based computing system 20008 may be used to monitor biomarkers associated with a surgeon or a patient in real-time and to generate surgical plans based at least on measurement data gathered prior to a surgical procedure, provide control signals to the surgical instruments during a surgical procedure, and notify a patient of a complication during post-surgical period.

The cloud-based computing system 20008 may be used to analyze surgical data. Surgical data may be obtained via one or more intelligent instrument(s) 20014, wearable sensing system(s) 20011, environmental sensing system(s) 20015, robotic system(s) 20013 and/or the like in the surgical system 20002. Surgical data may include, tissue states to assess leaks or perfusion of sealed tissue after a tissue sealing and cutting procedure pathology data, including images of samples of body tissue, anatomical structures of the body using a variety of sensors integrated with imaging devices and techniques such as overlaying images captured by multiple imaging devices, image data, and/or the like. The surgical data may be analyzed to improve surgical procedure outcomes by determining if further treatment, such as the application of endoscopic intervention, emerging technologies, a targeted radiation, targeted intervention, and precise robotics to tissue-specific sites and conditions. Such data analysis may employ outcome analytics processing and using standardized approaches may provide beneficial feedback to either confirm surgical treatments and the behavior of the surgeon or suggest modifications to surgical treatments and the behavior of the surgeon.

FIG. 2 shows an example surgical system 20002 in a surgical operating room. As illustrated in FIG. 2, a patient is being operated on by one or more HCPs. The HCPs are being monitored by one or more HCP sensing systems 20020 worn by the HCPs. The HCPs and the environment surrounding the HCPs may also be monitored by one or more environmental sensing systems including, for example, a set of cameras 20021, a set of microphones 20022, and other sensors that may be deployed in the operating room. The HCP sensing systems 20020 and the environmental sensing systems may be in communication with a surgical hub 20006, which in turn may be in communication with one or more cloud servers 20009 of the cloud computing system 20008, as shown in FIG. 1. The environmental sensing systems may be used for measuring one or more environmental attributes, for example, HCP position in the surgical theater, HCP movements, ambient noise in the surgical theater, temperature/humidity in the surgical theater, etc.

As illustrated in FIG. 2, a primary display 20023 and one or more audio output devices (e.g., speakers 20019) are positioned in the sterile field to be visible to an operator at the operating table 20024. In addition, a visualization/notification tower 20026 is positioned outside the sterile field. The visualization/notification tower 20026 may include a first non-sterile human interactive device (HID) 20027 and a second non-sterile HID 20029, which may face away from each other. The HID may be a display or a display with a touchscreen allowing a human to interface directly with the HID. A human interface system, guided by the surgical hub 20006, may be configured to utilize the HIDs 20027, 20029, and 20023 to coordinate information flow to operators inside and outside the sterile field. In an example, the surgical hub 20006 may cause an HID (e.g., the primary HID 20023) to display a notification and/or information about the patient and/or a surgical procedure step. In an example, the surgical hub 20006 may prompt for and/or receive input from personnel in the sterile field or in the non-sterile area. In an example, the surgical hub 20006 may cause an HID to display a snapshot of a surgical site, as recorded by an imaging device 20030, on a non-sterile HID 20027 or 20029, while maintaining a live feed of the surgical site on the primary HID 20023. The snapshot on the non-sterile display 20027 or 20029 can permit a non-sterile operator to perform a diagnostic step relevant to the surgical procedure, for example.

The surgical hub 20006 may be configured to route a diagnostic input or feedback entered by a non-sterile operator at the visualization tower 20026 to the primary display 20023 within the sterile field, where it can be viewed by a sterile operator at the operating table. In an example, the input can be in the form of a modification to the snapshot displayed on the non-sterile display 20027 or 20029, which can be routed to the primary display 20023 by the surgical hub 20006.

Referring to FIG. 2, a surgical instrument 20031 is being used in the surgical procedure as part of the surgical system 20002. The hub 20006 may be configured to coordinate information flow to a display of the surgical instrument(s) 20031. For example, in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety. A diagnostic input or feedback entered by a non-sterile operator at the visualization tower 20026 can be routed by the hub 20006 to the surgical instrument display within the sterile field, where it can be viewed by the operator of the surgical instrument 20031. Example surgical instruments that are suitable for use with the surgical system 20002 are described under the heading "Surgical Instrument Hardware" and in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety, for example.

As shown in FIG. 2, the surgical system 20002 can be used to perform a surgical procedure on a patient who is lying down on an operating table 20024 in a surgical operating room 20035. A robotic system 20034 may be used in the surgical procedure as a part of the surgical system 20002. The robotic system 20034 may include a surgeon's console 20036, a patient side cart 20032 (surgical robot), and a surgical robotic hub 20033. The patient side cart 20032 can manipulate at least one removably coupled surgical tool 20037 through a minimally invasive incision in the body of the patient while the surgeon views the surgical site through the surgeon's console 20036. An image of the surgical site can be obtained by a medical imaging device 20030, which can be manipulated by the patient side cart 20032 to orient the imaging device 20030. The robotic hub 20033 can be used to process the images of the surgical site for subsequent display to the surgeon through the surgeon's console 20036.

Other types of robotic systems can be readily adapted for use with the surgical system 20002. Various examples of robotic systems and surgical tools that are suitable for use with the present disclosure are described herein, as well as in U.S. Patent Application Publication No. US 2019-0201137 A1 (U.S. Patent Application No. 16/209,407), titled METHOD OF ROBOTIC HUB COMMUNICATION, DETECTION, AND CONTROL, filed December 4, 2018, the disclosure of which is herein incorporated by reference in its entirety.

In various aspects, the imaging device 20030 may include at least one image sensor and one or more optical components. Suitable image sensors may include, but are not limited to, Charge-Coupled Device (CCD) sensors and Complementary Metal-Oxide Semiconductor (CMOS) sensors.

The optical components of the imaging device 20030 may include one or more illumination sources and/or one or more lenses. The one or more illumination sources may be directed to illuminate portions of the surgical field. The one or more image sensors may receive light reflected or refracted from the surgical field, including light reflected or refracted from tissue and/or surgical instruments.

The illumination source(s) may be configured to radiate electromagnetic energy in the visible spectrum as well as the invisible spectrum. The visible spectrum, sometimes referred to as the optical spectrum or luminous spectrum, is the portion of the electromagnetic spectrum that is visible to (e.g., can be detected by) the human eye and may be referred to as visible light or simply light. A typical human eye will respond to wavelengths in air that range from about 380 nm to about 750 nm.

The invisible spectrum (e.g., the non-luminous spectrum) is the portion of the electromagnetic spectrum that lies below and above the visible spectrum (i.e., wavelengths below about 380 nm and above about 750 nm). The invisible spectrum is not detectable by the human eye. Wavelengths greater than about 750 nm are longer than the red visible spectrum, and they become invisible infrared (IR), microwave, and radio electromagnetic radiation. Wavelengths less than about 380 nm are shorter than the violet spectrum, and they become invisible ultraviolet, x-ray, and gamma ray electromagnetic radiation.

In various aspects, the imaging device 20030 is configured for use in a minimally invasive procedure. Examples of imaging devices suitable for use with the present disclosure include, but are not limited to, an arthroscope, angioscope, bronchoscope, choledochoscope, colonoscope, cytoscope, duodenoscope, enteroscope, esophagogastro-duodenoscope (gastroscope), endoscope, laryngoscope, nasopharyngo-neproscope, sigmoidoscope, thoracoscope, and ureteroscope.

The imaging device may employ multi-spectrum monitoring to discriminate topography and underlying structures. A multi-spectral image is one that captures image data within specific wavelength ranges across the electromagnetic spectrum. The wavelengths may be separated by filters or by the use of instruments that are sensitive to particular wavelengths, including light from frequencies beyond the visible light range, e.g., IR and ultraviolet. Spectral imaging can allow extraction of additional information that the human eye fails to capture with its receptors for red, green, and blue. The use of multi-spectral imaging is described in greater detail under the heading "Advanced Imaging Acquisition Module" in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety. Multi-spectrum monitoring can be a useful tool in relocating a surgical field after a surgical task is completed to perform one or more of the previously described tests on the treated tissue. It is axiomatic that strict sterilization of the operating room and surgical equipment is required during any surgery. The strict hygiene and sterilization conditions required in a "surgical theater," e.g., an operating or treatment room, necessitate the highest possible sterility of all medical devices and equipment. Part of that sterilization process is the need to sterilize anything that comes in contact with the patient or penetrates the sterile field, including the imaging device 20030 and its attachments and components. It will be appreciated that the sterile field may be considered a specified area, such as within a tray or on a sterile towel, that is considered free of microorganisms, or the sterile field may be considered an area, immediately around a patient, who has been prepared for a surgical procedure. The sterile field may include the scrubbed team members, who are properly attired, and all furniture and fixtures in the area.

Wearable sensing system 20011 illustrated in FIG. 1 may include one or more HCP sensing systems 20020 as shown in FIG. 2. The HCP sensing systems 20020 may include sensing systems to monitor and detect a set of physical states and/or a set of physiological states of a HCP. An HCP may be a surgeon or one or more healthcare personnel assisting the surgeon or other healthcare service providers in general. In an example, an HCP sensing system 20020 may measure a set of biomarkers to monitor the heart rate of an HCP. In an example, an HCP sensing system 20020 worn on a surgeon's wrist (e.g., a watch or a wristband) may use an accelerometer to detect hand motion and/or shakes and determine the magnitude and frequency of tremors. The sensing system 20020 may send the measurement data associated with the set of biomarkers and the data associated with a physical state of the surgeon to the surgical hub 20006 for further processing.

The environmental sensing system(s) 20015 shown in FIG. 1 may send environmental information to the surgical hub 20006. For example, the environmental sensing system(s) 20015 may include a camera 20021 for detecting hand/body position of an HCP. The environmental sensing system(s) 20015 may include microphones 20022 for measuring the ambient noise in the surgical theater. Other environmental sensing system(s) 20015 may include devices, for example, a thermometer to measure temperature and a hygrometer to measure humidity of the surroundings in the surgical theater, etc. The surgeon biomarkers may include one or more of the following: stress, heart rate, etc. The environmental measurements from the surgical theater may include ambient noise level associated with the surgeon or the patient, surgeon and/or staff movements, surgeon and/or staff attention level, etc. The surgical hub 20006, alone or in communication with the cloud computing system, may use the surgeon biomarker measurement data and/or environmental sensing information to modify the control algorithms of hand-held instruments or the averaging delay of a robotic interface, for example, to minimize tremors.

The surgical hub 20006 may use the surgeon biomarker measurement data associated with an HCP to adaptively control one or more surgical instruments 20031. For example, the surgical hub 20006 may send a control program to a surgical instrument 20031 to control its actuators to limit or compensate for fatigue and use of fine motor skills. The surgical hub 20006 may send the control program based on situational awareness and/or the context on importance or criticality of a task. The control program may instruct the instrument to alter operation to provide more control when control is needed.

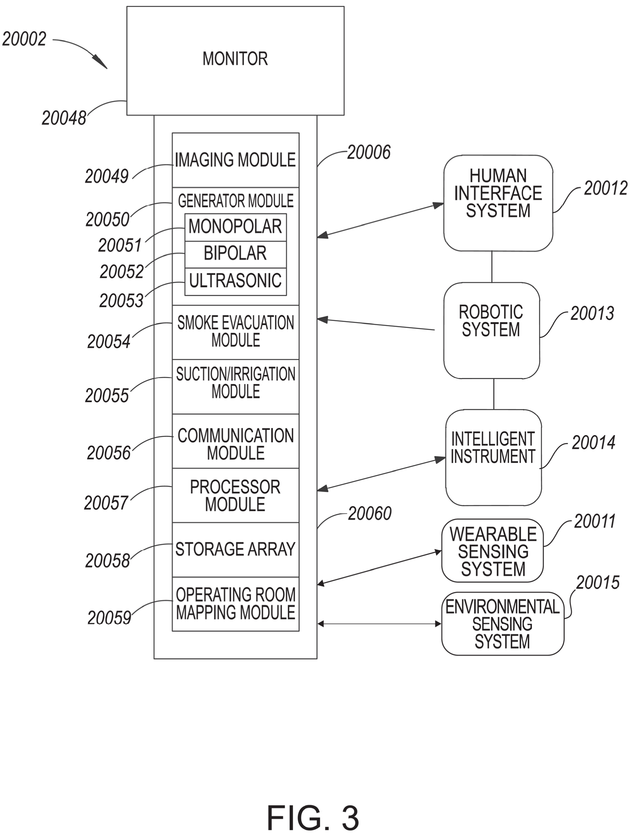

FIG. 3 shows an example surgical system 20002 with a surgical hub 20006. The surgical hub 20006 may be paired with, via a modular control, a wearable sensing system 20011, an environmental sensing system 20015, a human interface system 20012, a robotic system 20013, and an intelligent instrument 20014. The hub 20006 includes a display 20048, an imaging module 20049, a generator module 20050 (e.g., an energy generator), a communication module 20056, a processor module 20057, a storage array 20058, and an operating-room mapping module 20059. In certain aspects, as illustrated in FIG. 3, the hub 20006 further includes a smoke evacuation module 20054 and/or a suction/irrigation module 20055. The various modules and systems may be connected to the modular control either directly via a router or via the communication module 20056. The operating theater devices may be coupled to cloud computing resources and data storage via the modular control. The human interface system 20012 may include a display sub-system and a notification sub-system.

The modular control may be coupled to non-contact sensor module. The non-contact sensor module may measure the dimensions of the operating theater and generate a map of the surgical theater using ultrasonic, laser-type, and/or the like, non-contact measurement devices. Other distance sensors can be employed to determine the bounds of an operating room. An ultrasound-based non-contact sensor module may scan the operating theater by transmitting a burst of ultrasound and receiving the echo when it bounces off the perimeter walls of an operating theater as described under the heading "Surgical Hub Spatial Awareness Within an Operating Room" in U.S. Provisional Patent Application Serial No. 62/611,341, titled INTERACTIVE SURGICAL PLATFORM, filed December 28, 2017, which is herein incorporated by reference in its entirety. The sensor module may be configured to determine the size of the operating theater and to adjust Bluetooth-pairing distance limits. A laser-based non-contact sensor module may scan the operating theater by transmitting laser light pulses, receiving laser light pulses that bounce off the perimeter walls of the operating theater, and comparing the phase of the transmitted pulse to the received pulse to determine the size of the operating theater and to adjust Bluetooth pairing distance limits, for example.

During a surgical procedure, energy application to tissue, for sealing and/or cutting, may be associated with smoke evacuation, suction of excess fluid, and/or irrigation of the tissue. Fluid, power, and/or data lines from different sources may be entangled during the surgical procedure. Valuable time can be lost addressing this issue during a surgical procedure. Detangling the lines may necessitate disconnecting the lines from their respective modules, which may require resetting the modules. The hub modular enclosure 20060 may offer a unified environment for managing the power, data, and fluid lines, which reduces the frequency of entanglement between such lines.

Energy may be applied to tissue at a surgical site. The surgical hub 20006 may include a hub enclosure 20060 and a combo generator module slidably receivable in a docking station of the hub enclosure 20060. The docking station may include data and power contacts. The combo generator module may include two or more of: an ultrasonic energy generator component, a bipolar RF energy generator component, or a monopolar RF energy generator component that are housed in a single unit. The combo generator module may include a smoke evacuation component, at least one energy delivery cable for connecting the combo generator module to a surgical instrument, at least one smoke evacuation component configured to evacuate smoke, fluid, and/or particulates generated by the application of therapeutic energy to the tissue, and a fluid line extending from the remote surgical site to the smoke evacuation component. The fluid line may be a first fluid line, and a second fluid line may extend from the remote surgical site to a suction and irrigation module 20055 slidably received in the hub enclosure 20060. The hub enclosure 20060 may include a fluid interface.

The combo generator module may generate multiple energy types for application to the tissue. One energy type may be more beneficial for cutting the tissue, while another different energy type may be more beneficial for sealing the tissue. For example, a bipolar generator can be used to seal the tissue while an ultrasonic generator can be used to cut the sealed tissue. Aspects of the present disclosure present a solution where a hub modular enclosure 20060 is configured to accommodate different generators and facilitate an interactive communication therebetween. The hub modular enclosure 20060 may enable the quick removal and/or replacement of various modules.

The modular surgical enclosure may include a first energy-generator module, configured to generate a first energy for application to the tissue, and a first docking station comprising a first docking port that includes first data and power contacts, wherein the first energy-generator module is slidably movable into an electrical engagement with the power and data contacts and wherein the first energy-generator module is slidably movable out of the electrical engagement with the first power and data contacts. The modular surgical enclosure may include a second energy-generator module configured to generate a second energy, different than the first energy, for application to the tissue, and a second docking station comprising a second docking port that includes second data and power contacts, wherein the second energy generator module is slidably movable into an electrical engagement with the power and data contacts, and wherein the second energy-generator module is slidably movable out of the electrical engagement with the second power and data contacts. In addition, the modular surgical enclosure also includes a communication bus between the first docking port and the second docking port, configured to facilitate communication between the first energy-generator module and the second energy-generator module.

Referring to FIG. 3, the hub modular enclosure 20060 may allow the modular integration of a generator module 20050, a smoke evacuation module 20054, and a suction/irrigation module 20055. The hub modular enclosure 20060 may facilitate interactive communication between the modules 20059, 20054, and 20055. The generator module 20050 can be with integrated monopolar, bipolar, and ultrasonic components supported in a single housing unit slidably insertable into the hub modular enclosure 20060. The generator module 20050 may connect to a monopolar device 20051, a bipolar device 20052, and an ultrasonic device 20053. The generator module 20050 may include a series of monopolar, bipolar, and/or ultrasonic generator modules that interact through the hub modular enclosure 20060. The hub modular enclosure 20060 may facilitate the insertion of multiple generators and interactive communication between the generators docked into the hub modular enclosure 20060 so that the generators would act as a single generator.

A surgical data network having a set of communication hubs may connect the sensing system(s), the modular devices located in one or more operating theaters of a healthcare facility, a patient recovery room, or a room in a healthcare facility specially equipped for surgical operations, to the cloud computing system 20008.

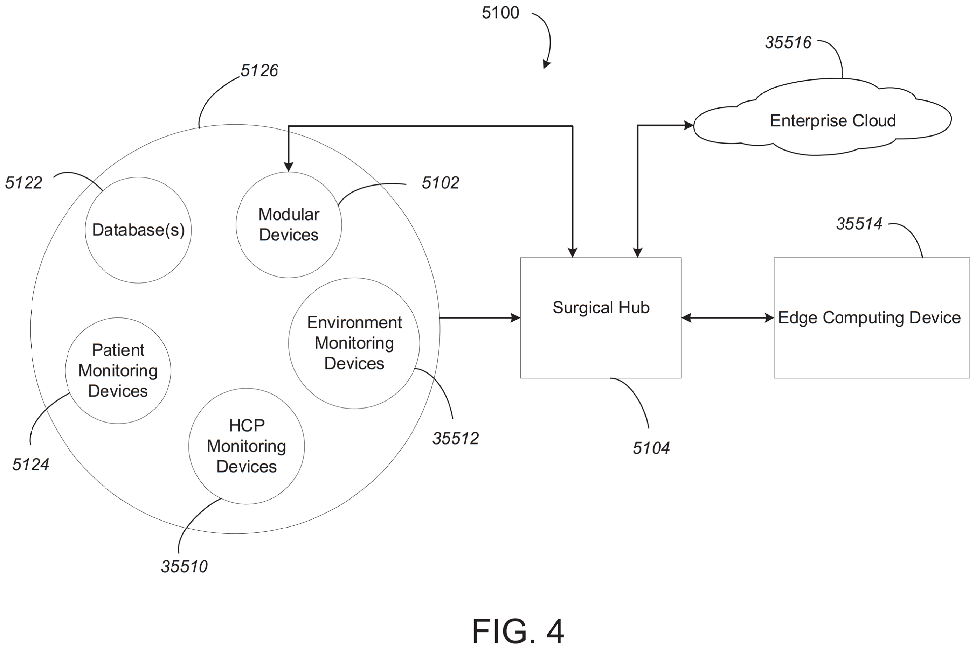

FIG. 4 illustrates a diagram of a situationally aware surgical system 5100. The data sources 5126 may include, for example, the modular devices 5102, databases 5122 (e.g., an EMR database containing patient records), patient monitoring devices 5124 (e.g., a blood pressure (BP) monitor and an electrocardiography (EKG) monitor), HCP monitoring devices 35510, and/or environment monitoring devices 35512. The modular devices 5102 may include sensors configured to detect parameters associated with the patient, HCPs and environment and/or the modular device itself. The modular devices 5102 may include one or more intelligent instrument(s) 20014. The surgical hub 5104 may derive the contextual information pertaining to the surgical procedure from the data based upon, for example, the particular combination(s) of received data or the particular order in which the data is received from the data sources 5126. The contextual information inferred from the received data can include, for example, the type of surgical procedure being performed, the particular step of the surgical procedure that the surgeon is performing, the type of tissue being operated on, or the body cavity that is the subject of the procedure. This ability by some aspects of the surgical hub 5104 to derive or infer information related to the surgical procedure from received data can be referred to as "situational awareness." For example, the surgical hub 5104 can incorporate a situational awareness system, which may be the hardware and/or programming associated with the surgical hub 5104 that derives contextual information pertaining to the surgical procedure from the received data and/or a surgical plan information received from the edge computing system 35514 or an enterprise cloud server 35516. The contextual information derived from the data sources 5126 may include, for example, what step of the surgical procedure is being performed, whether and how a particular modular device 5102 is being used, and the patient's condition.

The surgical hub 5104 may be connected to various databases 5122 to retrieve therefrom data regarding the surgical procedure that is being performed or is to be performed. In one exemplification of the surgical system 5100, the databases 5122 may include an EMR database of a hospital. The data that may be received by the situational awareness system of the surgical hub 5104 from the databases 5122 may include, for example, start (or setup) time or operational information regarding the procedure (e.g., a segmentectomy in the upper right portion of the thoracic cavity). The surgical hub 5104 may derive contextual information regarding the surgical procedure from this data alone or from the combination of this data and data from other data sources 5126.

The surgical hub 5104 may be connected to (e.g., paired with) a variety of patient monitoring devices 5124. In an example of the surgical system 5100, the patient monitoring devices 5124 that can be paired with the surgical hub 5104 may include a pulse oximeter (SpO2 monitor) 5114, a BP monitor 5116, and an EKG monitor 5120. The perioperative data that is received by the situational awareness system of the surgical hub 5104 from the patient monitoring devices 5124 may include, for example, the patient's oxygen saturation, blood pressure, heart rate, and other physiological parameters. The contextual information that may be derived by the surgical hub 5104 from the perioperative data transmitted by the patient moni-toring devices 5124 may include, for example, whether the patient is located in the operating theater or under anesthesia. The surgical hub 5104 may derive these inferences from data from the patient monitoring devices 5124 alone or in combination with data from other data sources 5126 (e.g., the ventilator 5118).

The surgical hub 5104 may be connected to (e.g., paired with) a variety of modular devices 5102. In one exemplification of the surgical system 5100, the modular devices 5102 that are paired with the surgical hub 5104 may include a smoke evacuator, a medical imaging device such as the imaging device 20030 shown in FIG. 2, an insufflator, a combined energy generator (for powering an ultrasonic surgical instrument and/or an RF electrosurgical instrument), and a ventilator.

The perioperative data received by the surgical hub 5104 from the medical imaging device may include, for example, whether the medical imaging device is activated and a video or image feed. The contextual information that is derived by the surgical hub 5104 from the perioperative data sent by the medical imaging device may include, for example, whether the procedure is a VATS procedure (based on whether the medical imaging device is activated or paired to the surgical hub 5104 at the beginning or during the course of the procedure). The image or video data from the medical imaging device (or the data stream (e.g., dataflow) representing the video for a digital medical imaging device) may be processed by a pattern recognition system or a ML system to recognize features (e.g., organs or tissue types) in the field of view (FOY) of the medical imaging device, for example. The contextual information that is derived by the surgical hub 5104 from the recognized features may include, for example, what type of surgical procedure (or step thereof) is being performed, what organ is being operated on, or what body cavity is being operated in.

The situational awareness system of the surgical hub 5104 may derive the contextual information from the data received from the data sources 5126 in a variety of different ways. For example, the situational awareness system can include a pattern recognition system, or ML system (e.g., an artificial neural network), that has been trained on training data to correlate various inputs (e.g., data from database(s) 5122, patient monitoring devices 5124, modular devices 5102, HCP monitoring devices 35510, and/or environment monitoring devices 35512) to corresponding contextual information regarding a surgical procedure. For example, a ML system may accurately derive contextual information regarding a surgical procedure from the provided inputs. In examples, the situational awareness system can include a LUT storing pre-characterized contextual information regarding a surgical procedure in association with one or more inputs (or ranges of inputs) corresponding to the contextual information. In response to a query with one or more inputs, the LUT can return the corresponding contextual information for the situational awareness system for controlling the modular devices 5102. In examples, the contextual information received by the situational awareness system of the surgical hub 5104 can be associated with a particular control adjustment or set of control adjustments for one or more modular devices 5102. In examples, the situational awareness system can include a ML system, LUT, or other such system, which may generate or retrieve one or more control adjustments for one or more modular devices 5102 when provided the contextual information as input.

For example, based on the data sources 5126, the situationally aware surgical hub 5104 may determine what type of tissue was being operated on. The situationally aware surgical hub 5104 can infer whether a surgical procedure being performed is a thoracic or an abdominal procedure, allowing the surgical hub 5104 to determine whether the tissue clamped by an end effector of the surgical stapling and cutting instrument is lung (for a thoracic procedure) or stomach (for an abdominal procedure) tissue. The situationally aware surgical hub 5104 may determine whether the surgical site is under pressure (by determining that the surgical procedure is utilizing insufflation) and determine the procedure type, for a consistent amount of smoke evacuation for both thoracic and abdominal procedures. Based on the data sources 5126, the situationally aware surgical hub 5104 could determine what step of the surgical procedure is being performed or will subsequently be performed.

The situationally aware surgical hub 5104 could determine what type of surgical procedure is being performed and customize the energy level according to the expected tissue profile for the surgical procedure. The situationally aware surgical hub 5104 may adjust the energy level for the ultrasonic surgical instrument or RF electrosurgical instrument throughout the course of a surgical procedure, rather than just on a procedure-by-procedure basis.

In examples, data can be drawn from additional data sources 5126 to improve the conclusions that the surgical hub 5104 draws from one data source 5126. The situationally aware surgical hub 5104 could augment data that it receives from the modular devices 5102 with contextual information that it has built up regarding the surgical procedure from other data sources 5126.

The situational awareness system of the surgical hub 5104 can consider the physiological measurement data to provide additional context in analyzing the visualization data. The additional context can be useful when the visualization data may be inconclusive or incomplete on its own.

The situationally aware surgical hub 5104 could determine whether the surgeon (or other HCP(s)) was making an error or otherwise deviating from the expected course of action during the course of a surgical procedure. For example, the surgical hub 5104 may determine the type of surgical procedure being performed, retrieve the corresponding list of steps or order of equipment usage (e.g., from a memory), and compare the steps being performed or the equipment being used during the course of the surgical procedure to the expected steps or equipment for the type of surgical procedure that the surgical hub 5104 determined is being performed. The surgical hub 5104 can provide an alert indicating that an unexpected action is being performed or an unexpected device is being utilized at the particular step in the surgical procedure.

The surgical instruments (and other modular devices 5102) may be adjusted for the particular context of each surgical procedure (such as adjusting to different tissue types) and validating actions during a surgical procedure. Next steps, data, and display adjustments may be provided to surgical instruments (and other modular devices 5102) in the surgical theater according to the specific context of the procedure.

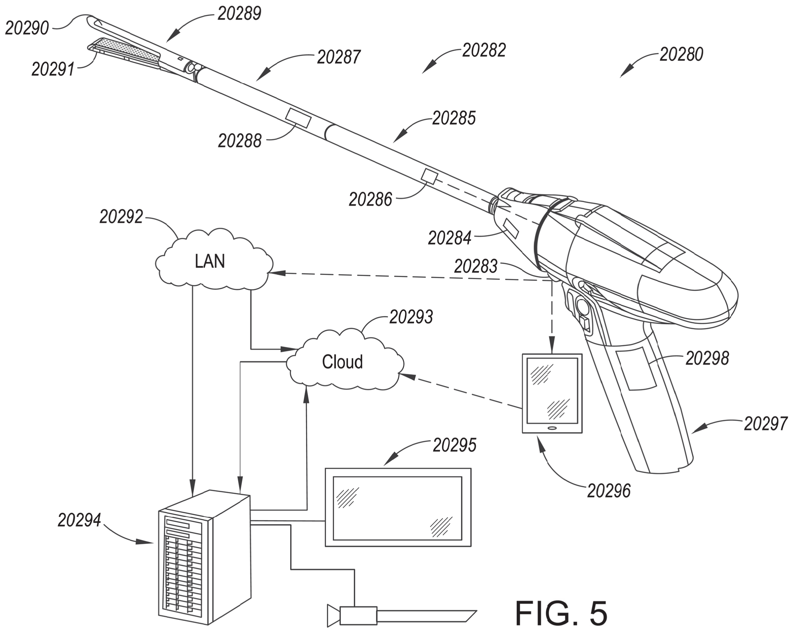

FIG. 5 illustrates an example surgical system 20280 that may include a surgical instrument 20282. The surgical instrument 20282 can be in communication with a console 20294 and/or a portable device 20296 through a local area network 20292 and/or a cloud network 20293 via a wired and/or wireless connection. The console 20294 and the portable device 20296 may be any suitable computing device. Surgical instrument 20282 may include a handle 20297, an adapter 20285, and a loading unit 20287. The adapter 20285 releasably couples to the handle 20297 and the loading unit 20287 releasably couples to the adapter 20285 such that the adapter 20285 transmits a force from a drive shaft to the loading unit 20287. The adapter 20285 or the loading unit 20287 may include a force gauge (not explicitly shown) disposed therein to measure a force exerted on the loading unit 20287. The loading unit 20287 may include an end effector 20289 having a first jaw 20291 and a second jaw 20290. The loading unit 20287 may be an in-situ loaded or multi-firing loading unit (MFLU) that allows a clinician to fire a plurality of fasteners multiple times without requiring the loading unit 20287 to be removed from a surgical site to reload the loading unit 20287.

The first and second jaws 20291, 20290 may be configured to clamp tissue therebetween, fire fasteners through the clamped tissue, and sever the clamped tissue. The first jaw 20291 may be configured to fire at least one fastener a plurality of times or may be configured to include a replaceable multi-fire fastener cartridge including a plurality of fasteners (e.g., staples, clips, etc.) that may be fired more than one time prior to being replaced. The second jaw 20290 may include an anvil that deforms or otherwise secures the fasteners, as the fasteners are ejected from the multi-fire fastener cartridge.

The handle 20297 may include a motor that is coupled to the drive shaft to affect rotation of the drive shaft. The handle 20297 may include a control interface to selectively activate the motor. The control interface may include buttons, switches, levers, sliders, touchscreens, and any other suitable input mechanisms or user interfaces, which can be engaged by a clinician to activate the motor.

The control interface of the handle 20297 may be in communication with a controller 20298 of the handle 20297 to selectively activate the motor to affect rotation of the drive shafts. The controller 20298 may be disposed within the handle 20297 and may be configured to receive input from the control interface and adapter data from the adapter 20285 or loading unit data from the loading unit 20287. The controller 20298 may analyze the input from the control interface and the data received from the adapter 20285 and/or loading unit 20287 to selectively activate the motor. The handle 20297 may also include a display that is viewable by a clinician during use of the handle 20297. The display may be configured to display portions of the adapter or loading unit data before, during, or after firing of the instrument 20282.

The adapter 20285 may include an adapter identification device 20284 disposed therein and the loading unit 20287 may include a loading unit identification device 20288 disposed therein. The adapter identification device 20284 may be in communication with the controller 20298, and the loading unit identification device 20288 may be in communication with the controller 20298. It will be appreciated that the loading unit identification device 20288 may be in communication with the adapter identification device 20284, which relays or passes communication from the loading unit identification device 20288 to the controller 20298.

The adapter 20285 may also include a plurality of sensors 20286 (one shown) disposed thereabout to detect various conditions of the adapter 20285 or of the environment (e.g., if the adapter 20285 is connected to a loading unit, if the adapter 20285 is connected to a handle, if the drive shafts are rotating, the torque of the drive shafts, the strain of the drive shafts, the temperature within the adapter 20285, a number of firings of the adapter 20285, a peak force of the adapter 20285 during firing, a total amount of force applied to the adapter 20285, a peak retraction force of the adapter 20285, a number of pauses of the adapter 20285 during firing, etc.). The plurality of sensors 20286 may provide an input to the adapter identification device 20284 in the form of data signals. The data signals of the plurality of sensors 20286 may be stored within or be used to update the adapter data stored within the adapter identification device 20284. The data signals of the plurality of sensors 20286 may be analog or digital. The plurality of sensors 20286 may include a force gauge to measure a force exerted on the loading unit 20287 during firing.

The handle 20297 and the adapter 20285 can be configured to interconnect the adapter identification device 20284 and the loading unit identification device 20288 with the controller 20298 via an electrical interface. The electrical interface may be a direct electrical interface (i.e., include electrical contacts that engage one another to transmit energy and signals therebetween). Additionally, or alternatively, the electrical interface may be a non-contact electrical interface to wirelessly transmit energy and signals therebetween (e.g., inductively transfer). It is also contemplated that the adapter identification device 20284 and the controller 20298 may be in wireless communication with one another via a wireless connection separate from the electrical interface.

The handle 20297 may include a transceiver 20283 that is configured to transmit instrument data from the controller 20298 to other components of the system 20280 (e.g., the LAN 20292, the cloud 20293, the console 20294, or the portable device 20296). The controller 20298 may also transmit instrument data and/or measurement data associated with one or more sensors 20286 to a surgical hub. The transceiver 20283 may receive data (e.g., cartridge data, loading unit data, adapter data, or other notifications) from the surgical hub 20270. The transceiver 20283 may receive data (e.g., cartridge data, loading unit data, or adapter data) from the other components of the system 20280. For example, the controller 20298 may transmit instrument data including a serial number of an attached adapter (e.g., adapter 20285) attached to the handle 20297, a serial number of a loading unit (e.g., loading unit 20287) attached to the adapter 20285, and a serial number of a multi-fire fastener cartridge loaded into the loading unit to the console 20294. Thereafter, the console 20294 may transmit data (e.g., cartridge data, loading unit data, or adapter data) associated with the attached cartridge, loading unit, and adapter, respectively, back to the controller 20298. The controller 20298 can display messages on the local instrument display or transmit the message, via transceiver 20283, to the console 20294 or the portable device 20296 to display the message on the display 20295 or portable device screen, respectively.

### Example Operational Environment to Rebalance Dataflows

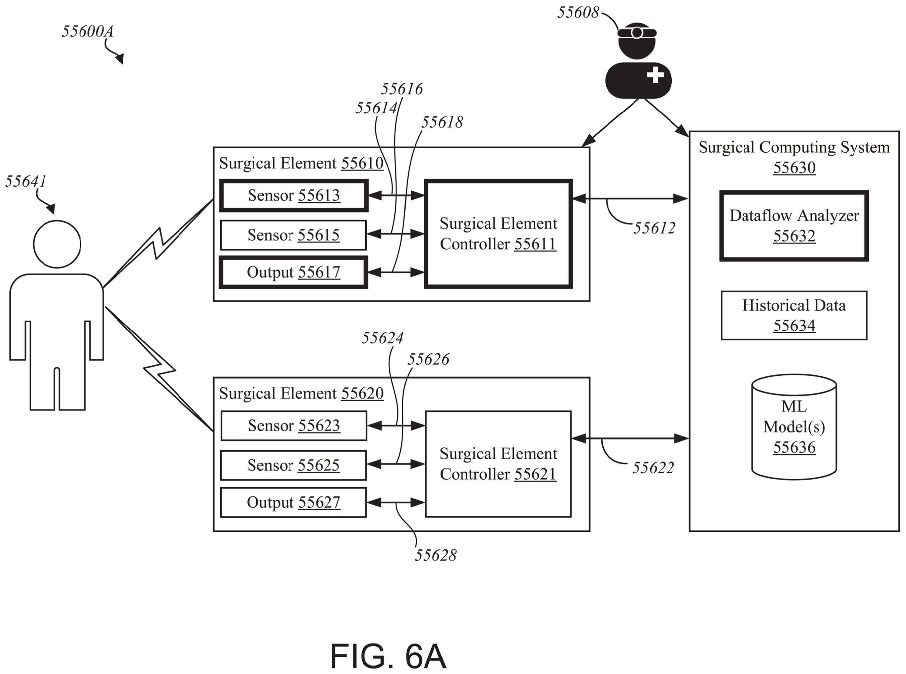

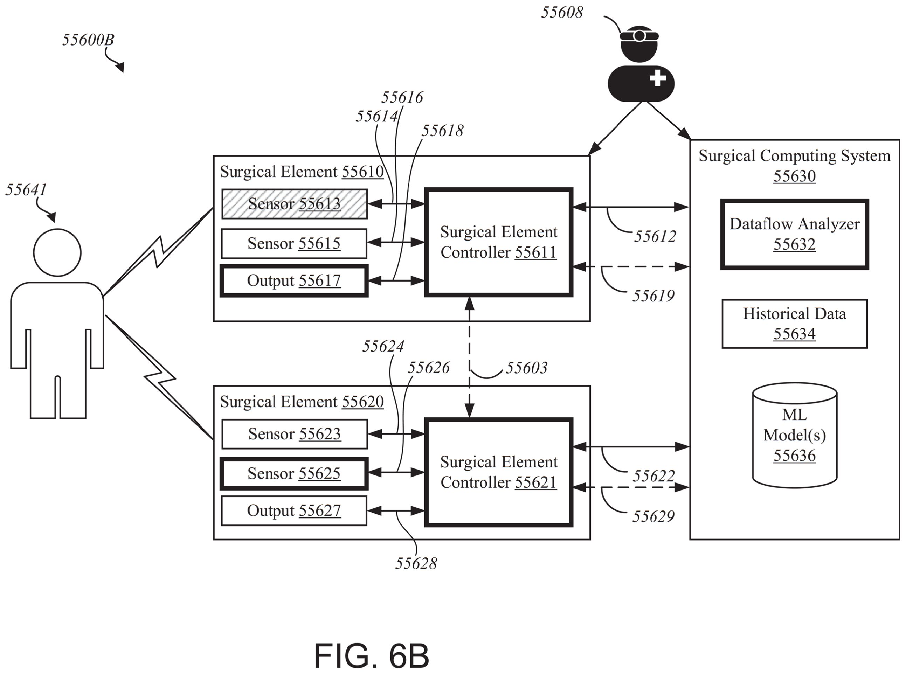

FIGS. 6A and 6B illustrate example operational environments 55600A, 55600B that may rebalance dataflows based on a number of unknowns. In examples, rebalancing dataflows based on the number of unknowns may include determining one or more dataflows to be added and/or included in a system to generate an optimized output and/or to prevent delay during a procedure. A dataflow may include sensor data, operational data, system generated data, control data, dataflow configuration information, and/or the like. The example operational environments 55600A, 55600B may include surgical element 55610, 55620, a patient 55641, a surgical computing system 55630, and/or an HCP 55608. Although operational environment 55600A, 55600B depict two surgical elements 55610, 55620, and/or one surgical computing system 55630, this is not intended to be limiting, as an example operational environment may include any combination of one or more surgical elements, surgical computing systems, HCPs, sensors, outputs, communication paths, and/or the like.

FIG. 6A illustrates an example operational environment 55600A (e.g., a typical control path as indicated with bold boarders). As part of operational environment 55600A, a surgical element 55610 (e.g., via sensor 55613) may provide sensor data (e.g., via 55614) to a surgical element controller 55611. The surgical element controller 55611 may provide a dataflow (e.g., via 55612) to a dataflow analyzer 55632. In response to receiving a dataflow, the dataflow analyzer 555632 may generate and/or send control data to the surgical element controller 55611 (e.g., via 55612). The surgical element controller 55611 may send the control data (e.g., via 55618) to an output 55617 (e.g., to cause the surgical element 55610 to adjust an output characteristic).

FIG. 6B illustrates an example operational environment 55600B (e.g., an alternative control path as indicated with bold boarders), where the surgical computing system 55630 may continue a procedure without the need to pause, cancel, and/or continue a procedure with a reduced number of surgical elements. The surgical computing system 55630 may continue a procedure without interruption by determining that a dataflow (e.g., via 55612) from failed sensor 55613 (e.g., as indicated with shading) is erroneous. The surgical computing system 55630 may communicate with a second surgical element 55620 (e.g., via 55622) to establish a second dataflow from sensor 55625. The surgical computing system 55630 may determine to communicate with a second surgical element 55620 based on sensor a determined relationship between sensor 55625 and/or sensor 55613. The second dataflow may be sent to the surgical computing system 55630 (e.g., via 55629) and/or sent to the surgical element 55610 (e.g., via 55603). The second dataflow may be used to determine control data, which may be sent (e.g., via 55619) to surgical element 55610 and/or determined by surgical element controller 55611. The control data may be sent to output 55617 (e.g., via 55618). Output 55617 may adjust an output characteristic of the surgical element 55610 based on the control data.

A surgical element 55610 may include sensor 55613, 55615, output 55617, and/or a surgical element controller 55611. Surgical element 55610 may be any one of a number of a instruments, devices, and/or the like, that may be present in an operating room during a procedure and/or capable of being connected (wired and/or wirelessly) to a surgical computing system 55630. In examples, surgical element 55610 may be a robotic surgical system, a navigation system, a smart imaging system, an endoscopic and/or laparoscopic system, a energy scalpel, an anesthesia machine, a patient monitoring system (pulse oximeters, blood pressure monitors, EKG monitors, EEG monitors, and/or the like), an energy device (e.g., electrosurgical units, laser surgery systems, and/or the like), an infusion pump, and/or the like.

A patient 55641 may be in communication with one or more surgical elements 55610, 55620 (e.g., sensor 55613, 55615, and/or output 55617, during a procedure). As an illustrative example, a ventilator (e.g., a surgical element 55610) and/or a pulse oximeter (e.g., surgical element 55620) may be attached to a patient during an open heart surgery. An HCP 55608 and/or a surgical computing system 55630 may interact with one or more surgical elements 55610, to monitor the oxygen saturation of the patient (e.g., via the pulse oximeter), and/or determine a flow rate for a ventilator. In examples, the HCP 55608 may interact with the surgical computing system 55630 and/or surgical elements 55610, 55620 via a user input (e.g., a graphical user interface (GUI), a knob, a button, a smart device a wearable electronic device, and/or the like).

Sensor 55613, 55615 may be any instrument, transducer, and/or the like, configured to measure one or more environmental conditions. In examples, sensor 55613, 55615, may measure physiological parameters of a patient 55641 such as oxygen saturation, blood pressure, respiratory rate, blood sugar, heart rate, a core body temperature and/or a hydration state and/or the like. Sensor 55613, 55615 may measure an environmental condition of an operating room (e.g., the operational environment 55600A, 55600B) such as a room temperature, the number of HCPs 55608 present during a procedure, the position of an HCP 55608 during a procedure, a humidity level, air quality, lighting levels, CO2 levels and/or the like. Sensor 55613, 55615 may measure one or more environmental conditions associated with a surgical element, such as a current, voltage, a pressure, a force applied, a distance, a vibration, an orientation, a flow rate, a status, and/or the like. As an illustrative example, an HVAC system may include a temperature, humidity, and/or air quality sensor, a robotic surgical system may include a force and/or pressure sensor, a depth sensor, a temperature sensor, a blood flow and/or oxygen sensor, a pH sensor (e.g., to measure blood gas), an electrocardiogram (ECG) sensor, a position sensor (e.g., to measure the position of a robotic arm), and/or the like.

Sensor 55613, 55615 may send sensor data (e.g., information associated with a measured environmental condition) to a surgical element controller 55611 (e.g., via 55614, 55616 respectively). Sensor data may be sent based on dataflow configuration information. Dataflow configuration information may include an indication of a surgical element ID, an indication whether a dataflow is available, an update and/or acknowledgement of dataflow configuration information, a unit of measure, a communication protocol (RS-232, Ethernet, TCP/IP, Bluetooth, and/or the like), scheduling and/or frequency information (e.g., a time and/or frequency that sensor data is to be sent), a destination (e.g., port information associated with the surgical element controller 556611), and/or security and/or access control credentials. In examples, an update and/or acknowledgement of dataflow configuration information may indicate that a second dataflow is available if a first dataflow is removed (e.g., taken off-line and/or replaced by the second dataflow on a configured channel), and/or that a second dataflow may be available if a first dataflow and a second dataflow share a channel.

Output 55617 may be any number of devices and/or instruments as part of a surgical element 55610 that may generate an output characteristic based on a received signal. In examples, output 55617 may include, among other components, motors and/or actuators (e.g., as part of a robotic device, a cutting tool, ventilators and/or the like), lighting output devices, GUls, cameras (e.g., as part of a borescope, IR camera, ultrasound camera, and/or the like), pumps (e.g., as part of smoke evacuation devices, suction devices, anesthesia delivery devices, IV infusion devices and/or the like), and/or lasers (e.g., as part of tissue ablation devices).

Output 55117 may receive control data from a surgical element controller 55611 (e.g., via 55618). In examples, output 55617 may adjust, based on control data received from a surgical element controller 55611, an output characteristic such as the speed of a cutting tool, the power intensity of an electrosurgical tool, a flow rate for an infusion pump, a position of a robotic arm, and/or the like. Although one output 55617 is included in surgical element 55610, it is to be understood that there may be multiple outputs, each receiving control data. As an illustrative example, a robotic system may include multiple outputs 55617 generating any number of outputs including positioning a robot, performing a measurement, capturing an image, creating an incision, and/or the like.

Surgical element 55610 may include a surgical element controller 55611. Surgical element controller may receive sensor data (e.g., via 55614, 55616) from sensor 55613, 55615. Surgical element controller 55611 may transmit control data to output 55617 (e.g., via 55618). A surgical element controller 55611 may communicate, a dataflow (e.g., sensor data, control data, dataflow configuration information, and/or the like) to/from a surgical computing system 55630. In examples, a surgical element controller 55611 may create and/or send a second dataflow (e.g., via 55619) to/from a surgical computing system 55630. In examples, surgical element controller 55611 may configure and/or send a dataflow based on a configuration message. In examples, a surgical element controller 55611 may determine that sensor data may be erroneous (e.g., as describe herein).

A surgical element controller 55611 may receive an interrogation message as described herein. In response, the surgical element controller 55611 may send an interrogation response to the surgical computing system 55630. The interrogation response may include the second dataflow and/or dataflow configuration information such as an indication whether a second dataflow is available an update and/or acknowledgement to dataflow configuration information, (e.g., such as an updated surgical element ID, an updated unit of measure and/or the like). In examples, the interrogation response may indicate that a surgical element 55610 may be configured to transmit a requested dataflow (e.g., that a dataflow is available, that one or more dataflows may be removed (e.g., taken off-line) to send the requested dataflow, and/or the like).

A surgical element controller 55611 may receive a configuration message as described herein. In response, the surgical element controller 55611 may send a configuration response to the surgical computing system 55630. The configuration response may indicate dataflow configuration information, and/or a dataflow of the surgical element 55610 (e.g., a dataflow associated with sensor 55613, 55615, and/or the like).

It is to be understood that surgical element 55620 and/or one or more associated components (e.g., sensor 55623, 55625, output 55627, and/or surgical element controller 55621) may include the same and/or similar interactions, features, functionalities, and/or the like, as described herein with reference to surgical element 55610 and/or one or more components of surgical element 55610 (e.g., sensor 55613, 55615, output 55617, surgical element controller 55611). Surgical element 55620 may be a second surgical element (e.g., separate from surgical element 55610). In examples, communication between one or more components as described with reference to surgical element 55610 may be the same and/or similar to one or more components of surgical element 55620. As an illustrative example, surgical element 55620 may send data to and/or receive data from a surgical computing system 55630 and/or a surgical element 55610, including an interrogation message, an interrogation response, a configuration message, a configuration response, control data, operational data, historical data, sensor data, a dataflow, system generated data, and/or the like (e.g., via 55622, 55624, 55626, 55628, 55629 and/or 55603).

In an illustrative example of two surgical elements as part of an operational environment, a first surgical element 55610 may be a laparoscopic tool including a temperature sensor, whereas a second surgical element 55620 may be a heating pad configured to heat an insufflated cavity. The laparoscopic tool may transmit a dataflow indicating a measured temperature of an insufflated cavity to the surgical computing system 55630, and/or the heating pad may transmit a measured temperature near an insufflated cavity to the surgical computing system 55630. In examples, the laparoscopic tool and/or the heating pad may have the same and/or different configuration information (e.g., the temperatures may not be transmitted at the same data rate, the temperature may not be in the same in the same units, a communication protocol may differ, a frequency of messages may be different, and/or the like).

Operational environment 55600A, 55600B may include a surgical computing system 55630. The surgical computing system 55630 may include a dataflow analyzer 55632, historical data 55634, and/or ML model(s) 55636.

A dataflow analyzer 55632 may include hardware, software, firmware and/or the like, to communicate with surgical element 55610, 55620, and/or an HCP 55608 (e.g., via a GUI), determine dataflow integrity (e.g., whether a dataflow is erroneous), establish relationships between surgical elements 55610, 55620, generate and/or store data (e.g., control data, historical data, sensor data, operational data, system generated data, training data, and/or the like).

A dataflow analyzer 55632 may receive a dataflow from a surgical element 55610, 55620 (e.g., via 55612, 55622 respectively). A dataflow may include sensor data, operational data, system generated data, control data, dataflow configuration information, and/or the like. In examples, dataflow analyzer 55632 may receive sensor data from sensor 55613. The sensor data may include a signal indicating a measurement of an environmental condition during a procedure (e.g., a current, voltage, a pressure, a force applied, a distance, a vibration, an orientation, a flow rate, a status, and/or the like). Sensor data may be configured (e.g., via surgical element controller 55611, 55621) based on dataflow configuration information as described herein. A dataflow analyzer 55632 may generate control data in response to receiving a dataflow. Control data may be sent to surgical element 55610, 55620, via 55612, 55622, to adjust an output characteristic of surgical element 55610, 55620.

Dataflow analyzer 55632 may determine and/or infer one or more relationships (e.g., a relational link) between components of an operational environment 55600A, 55600B. A relationship may be determined and/or inferred based on, for example, compatible sensors between surgical elements 55610, 55620, a determined dataflow that may be needed to resolve an issue during surgery, and/or based on a user input (e.g., by an HCP via a GUI). As an illustrative example, a dataflow analyzer 55632 may receive a first dataflow from a laparoscopic tool (e.g., a dataflow indicating a measured temperature of an insufflated cavity via sensor 55613) and a second dataflow from a heating pad (e.g., a dataflow indicating a measured temperature near an insufflated cavity via sensor 55625). The dataflow analyzer 55632 may determine, based on configuration information, sensor data, and/or control data associated with each dataflow, that the two dataflows are related (e.g., a change detected by a first temperature sensor of a second surgical element 55620 may be related to a response and/or a change in control data for a first surgical element 55610). After the dataflow analyzer 55632 determines that one or more aspects of surgical elements 55610, 55620 are related, the dataflow analyzer 55632 may store an indication of the relationship in, for example, historical data 55634 (e.g., as a LUT defining the one or more relationships). Additionally and/or alternatively, dataflow analyzer 55632 may determine and/or provide training data (e.g., including historical data 55634) to ML model(s) 55636, to train an ML model to determine one or more relationships.

A dataflow analyzer 55632 may determine the integrity of a dataflow (e.g., whether sensor data and/or the like, is erroneous). As described herein, erroneous data may be caused by, for example, an incorrectly calibrated sensor, a misplaced and/or improperly installed sensor (e.g., interference by a heat source), calibration drift, contamination, electromagnetic interference, a failed sensor, operator error, misalignment (e.g., in the case of a position sensor), and/or the like.

A dataflow analyzer 55632 may determine that a dataflow is erroneous, for example, if control data exceeds a threshold, based on a determination that the first dataflow is unavailable, a determination that the physiological parameter of the patient exceeds a patient safety threshold, based on a comparison of sensor data to historical data (e.g., past sensor data and/or another data type), and/or based on an output of ML model(s) 55636.

Control data may exceed a threshold if, for example, a signal indicates a higher and/or lower power output level than allowed by a surgical element and/or as indicated by an HCP 55608, a signal associated with control data does not change and/or changes too rapidly within an expected time, and/or the control data indicates incorrect configuration information as described herein. A first dataflow may be unavailable based on, for example, an indication received in an interrogation response and/or in a configuration response, and/or as indicated by an HCP 55608.

A physiological parameter of the patient may exceed a patient safety threshold if, for example, sensor data from a first sensor 55613 and/or a second sensor 55623 are not within a defined range. As an illustrative example, a dataflow analyzer 55632 may receive sensor data from multiple sensors (e.g., a heart rate and a raspatory rate), and determine that, during a procedure when a patient's heart rate increases, the patient's raspatory rate is expected to increase as well. If the raspatory rate does not increase based on a determined schedule, the dataflow analyzer 55632 may determine that one or more sensors are erroneous. In examples, one or more patient safety parameters may be utilized during a procedure (e.g., a first patient safety parameter may be monitored during administration of anesthesia, whereas a second patent safety parameter may be monitored during an incision).

A dataflow analyzer 55632 may determine that a dataflow is erroneous if the dataflow is coming from a source (e.g., a surgical element 55610, 55620) that is not a trusted source and/or trust may not be established. In examples, an untrusted source may be a surgical element 55610, 55620 that may not be associated with a relational link. In examples, a dataflow analyzer 55632 may introduce higher level functional, electrical, or mechanical constraints that, regardless of the dataflow source inputs, ensures the surgical computing system 55630 may not move outside of the higher level constraints (e.g., which may be correlated to physical safety, patient safety, device performance, and/or other intrinsic characteristics).

In examples, a dataflow analyzer 55632 may receive a user input from an HCP 55608 (e.g., via a GUI) to incorporate and/or identify dataflows. A dataflow analyzer 55632 may utilize untrusted data, and/or prompt the user (e.g., HCP 55608) for additional steps, constraints, and/or at multiple stages of a procedure to gain confidence, incorporate as part of training data, and/or reduce overall risk (e.g., by incorporating user feedback to mitigate the risk of the untrusted dataflow).

In examples, a dataflow analyzer 55632 may simulate potential risks associated with incorporation of an untrusted dataflow. A dataflow analyzer 55632 may execute a simulation and/or theoretical exercise of what may occur in the operational environment 55600A, 55600B, and/or to other articulation or handling limits if the dataflow analyzer 55632 were to incorporate the (e.g., new) dataflow. A dataflow analyzer 55632 may execute one or more simulations to anticipate the creation of any new additional risks.

A dataflow analyzer 55632 may be placed into a safe mode or safe space where the dataflow analyzer 55632 may incorporate the new untrusted data and/or test (including physical motion) how that data impacts and/or creates additional risks to the operational environment 55600A, 55600B.

A dataflow analyzer 55632 may monitor untrusted dataflows with relation to trusted dataflows (e.g., dataflows that may have an indicated relationship associated with the operational environment 55600A, 55600B). In examples, a dataflow analyzer 55632 may execute actions (e.g., such as moving an articulation joint or motor) and/or continue to monitor the untrusted data source, as well as other trusted sources that the dataflow analyzer 55632 has access to. The dataflow analyzer 55632 may correlate the performance of the untrusted dataflow to other trusted dataflows.

In examples, a dataflow analyzer 55632 may include one or more methods for handling inherently untrusted data. In examples, dataflow analyzer 55632 may execute a certificate inspection, a safety process, and/or intentionally induce latency and/or a limp mode (e.g., based on dataflow configuration information). In examples, a certificate inspection may include utilizing certificates, encryption keys, and/or other data trustworthiness mechanisms to ensure that data integrity of a dataflow has been preserved. In examples, a safety process may be executed after a trigger event. The safety process may monitor higher level functionality, such that the dataflow is coordinated. In examples, intentionally induced latency and/or a limp mode may include slowing down a speed associated with an output characteristic (e.g., associated with a dataflow) such that the user (e.g., an HCP 55608) may react if the output characteristic starts to misbehave and/or have a chance to correct based on user feedback (e.g., a user input via a GUI), as well as a chance to establish one or more relationships for the dataflow.

As described herein, the dataflow analyzer 55632 may determine to incorporate a second dataflow based on one or more relationships. If a (e.g., new) dataflow is found to be unreliable, the dataflow analyzer 55632 may choose to exclude the dataflow and/or default back to a prior dataflow.

In examples, a dataflow may include degrees of reliability. A dataflow may not be reliable (e.g., 100% reliable) to be used. As an illustrative example, a speed sensor on a piece of equipment may not be calibrated, and as a result, the speed sensor may output an incorrect speed. If the speed is incorrect, dataflow analyzer 55632 may use the dataflow to determine if a device is in motion and/or paused. In examples, erroneous dataflows may be linear and/or non-linear. A dataflow may be reliable within a zone of operation and/or unreliable in other zones (e.g., linearly offset in other zones).

In examples, a dataflow analyzer 55632 may determine the manner of an unreliable and/or dysfunctional dataflow. The manner of dysfunction or unreliability of the dataflow may impact the ability to utilize that dataflow in a capacity.

In examples, dataflow analyzer 55632 may determine the error of the dataflow. A secondary dataflow may be used to quantify an erroneous dataflow. As an illustrative example, a failure of a tachometer may indicate the incorrect speed of a fan. A dataflow analyzer 55632 may use other sensor data (e.g., such as for current or back-emf) to indicate the nature of the failed tachometer (e.g., miscalibration, complete failure, and/or the like).

In examples, a dataflow analyzer 55632 may utilize a known perturbation to determine the level of impact. As an illustrative example, a speed of a fan with a damaged tachometer may have its speed internationally increased to quantify the impact of the failure and/or understand if the tachometer has experienced a complete failure (e.g., no change with increased speed), offset failure, and/or calibration failure.

In examples, depending on the type of reliability issue (e.g., type of failure associated with an erroneous dataflow) with a dataflow, the dataflow may be used as a secondary verification rather than a primary control source (e.g., control with a second dataflow, and verify with a first dataflow). In examples, the first dataflow may be utilized at a (e.g., new) level with an indication, that if changes, may default back to an expected orientation.

A dataflow analyzer 55632 may replace an erroneous dataflow with a second dataflow (e.g., a second dataflow based on a determined relationship as described herein). As illustrated in FIG. 6B, sensor 55613 is shaded to indicate that the dataflow analyzer 55632 determined that a dataflow from sensor 55613 is erroneous. The dataflow analyzer 55632 may (e.g., based on one or more determined relationships) transmit an interrogation message and/or a configuration message to a second surgical element 55620, to establish a second dataflow to compensate for the erroneous data of the first dataflow. An example path of a second dataflow may be indicated by bold boarders associated with sensor 55625, surgical element controller 55621, dataflow analyzer 55632, surgical element controller 55611, and/or output 55617, and/or via 55629, 55619, and/or 55603 of operational environment 55600B. A second dataflow may be transmitted from surgical element 55620 to the dataflow analyzer 55632, and then to the surgical element 55610 (e.g., via 55629, 55619). If the second dataflow is transmitted to the dataflow analyzer 55632, the dataflow analyzer 55632 may use the second dataflow to generate control data for the first surgical element 55610 (e.g., to compensate for the erroneous sensor data of sensor 55613). Alternatively, a second dataflow transmitted from surgical element 55620 to a first surgical element 55610, (e.g., via 55603) may be used by the surgical element 55610 to determine control data associated with output 55617.

A dataflow analyzer 55632 may estimate sensor data associated with a failed sensor 55613. A dataflow analyzer 55632 may analyze control data (e.g., associated with surgical element 55610, 55620), sensor data (e.g., from sensor 55615, 55623, 55625), operational data, historical data, and/or system generated data, to estimate an environmental condition of an operational environment 55600A, 55600B.

A dataflow analyzer 55632 may be able to incorporate (e.g., new) dataflows of which the dataflow analyzer 55632 has knowledge that are available. A dataflow analyzer 55632 may identify available dataflows based on deterministic criteria. Deterministic criteria may include historical prior connections, dataflow status identification, searching, and/or listening. Deterministic criteria may be used in conjunction with and/or complementary to, one or more methods described herein for determining a dataflow.

A dataflow analyzer 55632 may determine a dataflow based on historical prior connections (e.g., as stored in historical data 55634). In examples, dataflow analyzer 55632 may be aware of prior connections (e.g., via historical data 55634, a user input from an HCP 55608, and/or the like), and as a result, utilize a dataflow based on the prior connection.

A dataflow analyzer 55632 may determine a dataflow based on a dataflow status ID (e.g., as part of dataflow configuration information). A dataflow status ID (e.g., a surgical element ID) may be included in metadata. A dataflow status ID may provide a status of one or more dataflows that may be made available (e.g., via communication with one or more surgical elements to allow the dataflow analyzer 55632 to understand one or more options for incorporation of the dataflow).

A dataflow analyzer 55632 may determine a dataflow based on a distributed data system. A distributed data system may include a list of topics available such as in the LUT described herein (e.g., similar to how a data distribution service network may be constructed). In examples, whether hardcoded or via communication, a data distribution service may have defined knowledge that one or more dataflows exist, and/or the dataflow analyzer 55632 may be able to then subscribe to one or more networks at a given time.

A dataflow analyzer 55632 may search for dataflows. In examples a dataflow analyzer 55632 may actively search (e.g., via an interrogation message described herein) across networks which the dataflow analyzer 55632 may have access to (e.g., wireless, electrical, and/or the like) to try to identify potential sources of information).

A dataflow analyzer 55632 may listen for dataflows. A dataflow analyzer 55632 may actively identify and/or announce that dataflows exist (e.g., such as with advertising data on a wireless beacon, and/or as a result, the dataflow analyzer 55632 may then identify and/or incorporate dataflows).

As described herein, the dataflow analyzer 55632 may transmit an interrogation message. An interrogation message may be sent by the dataflow analyzer 55632 to one or more surgical elements 55610, 55620, and/or to another surgical computing system. The interrogation message may be sent based on one or more determined relationships as described herein. In examples, the interrogation message may be sent by the dataflow analyzer 55632 to create, update, and/or verify one or more relational links (e.g., relationships) as described herein (e.g., with and/or without the detection of erroneous data). In examples, an interrogation message may be sent in response to a determination that a first dataflow is erroneous, based on a predefined frequency and/or time (e.g., as determined by the dataflow analyzer 55632), and/or based on a user input (e.g., by an HCP 55608 via a GUI). An interrogation message may, among other things, request confirmation that a second surgical element 55620 includes a compatible and/or available dataflow.

An interrogation message may include a request for information associated with one or more dataflows of a surgical element 55610, 55620. In examples, an interrogation message may request information associated with a second dataflow from a second surgical element 55620 (e.g., that may be related to a first dataflow of a first surgical element 55610). In examples, a dataflow analyzer 55632 may request, via the interrogation message, that the second surgical element 55620 may determine whether a second dataflow is available. The interrogation message may include information associated with a surgical element 55610, 55620 (e.g., to determine whether a second dataflow is available). In examples, information associated with a surgical element 55610, 55620, may include dataflow configuration information as described herein.

In examples, an interrogation message may be sent by the dataflow analyzer 55632 resolve an imbalance of a closed loop (e.g., to include a number of equations to balance a number of unknowns). As an illustrative example, a smoke evacuation system may be controlled by energy activation to prevent limitations of visibility. During the procedure, the HCP 55608 may be unable to see effectively (e.g., smoke evac may not effectively mitigate the issue). A scope may be added as a dataflow to control the smoke evacuation rate in the smoke evacuator. After the dataflow analyzer 55632 identifies an imbalance (e.g., identifies an erroneous dataflow), the dataflow analyzer 55632 may interact and/or search for a locally available dataflow to be used to resolve the imbalance (e.g., the dataflow analyzer 55632 may request, via an interrogation message, that advanced visualization system (e.g., a scope) provide a stream of occlusion magnitude, to look at the intake of the smoke evaluation for a particle count dataflow. The dataflow may be a particle type visualization (e.g., aerosol versus smoke), and/or an energy device jaw dataflow detection of tissue/body fluid interaction (e.g., whether the smoke evacuation system is immersed in blood, collagen tissue, and/or the like). The advanced visualization system may respond with an interrogation response, indicating a dataflow that may be used.

In response to sending an interrogation message, a dataflow analyzer 55632 may receive an interrogation response. An interrogation response may include dataflow configuration information, including an indication whether a second dataflow is available, an indication of an update to information associated with a surgical element 55610, 55620), and/or an acknowledgement of the information associated with the surgical element. An update to one or more determined relationships may include an indication of a second sensor that may be related to a failed sensor (e.g., sensor 55625 may be related to sensor 55613 in operational environment 55600B). In examples, the interrogation response may indicate that a surgical element 55610, 55620 may be configured to transmit the requested dataflow (e.g., that a dataflow is available, that one or more dataflows may be removed (e.g., taken off-line) to send the requested dataflow, and/or the like).

A dataflow analyzer 55632 may transmit a configuration message. A configuration message may be sent in response to a received interrogation response, based on a user input (e.g., an HCP 55608 via a GUI), and/or as determined by the dataflow analyzer 55632 (e.g., based on a determination that a dataflow is sending erroneous data and/or the like). A configuration message may request to configure a surgical element based on dataflow configuration information and/or a dataflow associated with a surgical element 55610, 55620. In examples, dataflow analyzer 55632 may instruct a second surgical element 55620 to transmit (e.g., via a configuration message and/or an interrogation message) a configuration response to a first surgical element 55610 (e.g., via 55603).

A configuration message may include a request for sensor data (e.g., as part of a dataflow) based on one or more relationships as determined by the dataflow analyzer 55632, ML model(s) 55636, and/or the like. A configuration message may include information associated with configuring a surgical element 55610, 55620 (e.g., dataflow configuration information). As an example, dataflow analyzer 55632 may send a configuration message to surgical element 55620, to configure a second dataflow (e.g., via 55622 and/or 55629) for receiving sensor data associated with sensor 55625. The configuration message may be sent based on a determination that sensor 55625 is related to failed sensor 55613 and/or that the dataflow analyzer 55632 may need a second dataflow to resolve an issue.

A dataflow analyzer 55632 may receive a configuration response. A configuration response may be received via 55612, 55619, 55622, and/or 55629. A configuration response may include dataflow configuration information, and/or a dataflow associated with a surgical element 55610, 55620. As an illustrative example, a configuration response may include a surgical element ID associated with surgical element 55620, a dataflow protocol associated with surgical element 55620 (e.g., ethernet and/or the like), the frequency that sensor data is sent to the dataflow analyzer 55632, and/or access control credentials associated with sensor 55625, surgical element 55620, and/or the like), and sensor data generated from sensor 55625 (e.g., to be used by the dataflow analyzer 55632 to generate control data for surgical element 55610).

A dataflow analyzer 55632 may utilize an arbitrator (e.g., not depicted as part of operational environment 55600A, 55600B) for connecting one or more surgical elements 55610, 55620. An arbitrator may enable bidirectional communication to be handled between surgical elements 55610, 55620, and/or a surgical computing system 55630 (e.g., may only support communication on a single bus).

In examples, surgical elements 55610, 55620, and/or a surgical computing system 55630 may not be configured for bussed and/or network communications, and/or may be intended for point to point communication (e.g., RS-232). In examples, a message (e.g., a dataflow, a configuration message, an interrogation message, and/or the like) that may be output from at least two surgical elements 55610, 55620, may be transmitted to an arbitrator before being received by the dataflow analyzer 55632. This methodology is not limited to RS-232, and may be employed for other communication modalities (e.g.,, including wired and/or wireless, Wi-Fi, and/or base station concepts to expand wireless networks). Use of an arbitrator can allow messages to be handled and output in sequential order, without creating message bus conflicts. An arbitrator may utilize any manner of embedded methodologies (e.g., interrupt driven, high speed implementation, and/or the like) to determine the order in which messages on one or more busses are dedicated. The order may become the internal order in which messages are passed through to the output of a surgical computing system 55630. To handle or mitigate potential overflows, messages may be briefly placed into memory such that message data is not lost.

A dataflow analyzer 55632 may generate control data based on a dataflow received from surgical element 55610, 55620, in response to a user input (e.g., by an HCP via a GUI), and/or as determined by the dataflow analyzer 55632. Control data may include information associated with, for example, a control variable setpoint (e.g., associated with a motor speed, a temperature, a flow rate, and/or the like), a current and/or voltage (e.g., associated with an electrosurgical tool, and/or the like), an instruction (e.g., to generate an image from a camera and/or the like), and/or a power level (e.g., of a heating pad, a laser ablation tool, and/or the like). As an illustrative example, control data may include a setpoint for the speed of a cutting tool, a voltage and/or current setpoint of an electrosurgical tool, a flow rate for an infusion pump, a position of a robotic arm, and/or the like.

A dataflow analyzer 55632 may transmit control data (e.g., via 55622, 55629) to a surgical element 55610, 55620, to cause the surgical element 55610, 55620, to adjust an output characteristic associated with output 55617, 55627. The output 55617, 55627, may adjust an output characteristic based on the information associated with a control variable setpoint, a current and/or voltage, an instruction, a power level, and/or the like, as indicated in the control data.

In examples, dataflow analyzer 55632 may instruct a second surgical element 55620 to transmit control data from the second surgical element 55620 to a first surgical element 55610 (e.g., via 55603). In examples, control data may be transmitted (e.g., via 55619) to adjust an output of a first surgical element 55610 based on a dataflow received from a second surgical element 55620 (e.g., as illustrated in operational environment 55600B).

A dataflow analyzer 55632 may generate control data based on multiple dataflows. A dataflow analyzer 55632 may, in response to a user input and/or as determined by the surgical computing system 55630, add a second dataflow to generate control data (e.g., without determining that a first dataflow is erroneous). As an illustrative example, an image camera (e.g., a first surgical element 55610) may transmit a first dataflow to a surgical computing system 55630 with a limited resolution. The limited resolution of the camera may not provide an HCP 55608 with a sufficient image during a procedure. The HCP 55608 may request, via a GUI, that the dataflow analyzer 55632 determine a second dataflow (e.g., based on one or more determined relationships between a first surgical element 55610 and a second surgical element 55620) that may be used with the first dataflow to enhance the resolution of the image. The dataflow analyzer 55632 may request a second dataflow (e.g., via a configuration message as described herein) from, for example, an IR camera (e.g., a second surgical element). The second dataflow may be utilized with the first dataflow to generate control data (e.g., to enhance the output image to the HCP 55608).

A dataflow analyzer 55632 may escalate a determination to include a second dataflow (e.g., whether an erroneous dataflow affects the safety of the patient) to an HCP 55608 and/or to the system 55630. In examples, the surgical computing system 55630 may be unable to generate control data based on one or more dataflows. The dataflow analyzer 55632 may determine that a second dataflow may be added to improve control data in the event that a first dataflow that is monitored, but not utilized, deviates from an expected range.

As an illustrative example, a smoke evacuation device may be controlled by energy activation to clear a field of view for an HCP 55608 during a procedure. If the dataflow analyzer 55632 determines that the smoke evacuation device is not effectively clearing the field of view, the dataflow analyzer 55632 may add a scope occlusion device (e.g., a dataflow analyzer 55632 may add a second dataflow from a scope occlusion device) to control the smoke evacuation rate (e.g., to adjust an output characteristic of the smoke evacuation device).

In examples, the second dataflow may already be monitored (e.g., received by the dataflow analyzer 55632), and/or the dataflow analyzer 55632 may include the existing dataflow from the additional device. As an illustrative example, a smoke evacuation device may be an advanced energy activation device (such as output 55617). A scope may measure a particle count to assess the effectiveness of the smoke evacuation device to clear the field of view. If the particle count is no longer successfully controlled, the dataflow analyzer 55632 may add an existing particle count dataflow from the scope to control smoke evacuation device.

In examples, the second dataflow may not be monitored (e.g., received by the dataflow analyzer 55632), and/or the dataflow analyzer 55632 may automatically include the new dataflow (e.g., according to FIGS. 6A, 6B, 7, 8, 9A, and/or 9B). As an illustrative example, a smoke evacuation device may be controlled by an advanced energy activation device (e.g., output 55617). An scope may measure a particle count to assess the effectiveness of the smoke evacuation device to clear the field of view. If the particle count is not successfully controlled (e.g., a threshold is reached and/or a trigger condition is satisfied), the dataflow analyzer 55632 may initiate infrared monitoring as a second dataflow on the scope to assess and/or monitor particle count. The dataflow analyzer 55632 may utilize the IR data to control the smoke evacuation device.

In examples, a second dataflow may not be monitored, and/or the dataflow analyzer 55632 may request a user input (e.g., via a GUI) to include the second dataflow. In examples, the dataflow analyzer 55632 may generate an indication (e.g., via a GUI) to the HCP 55608, informing the HCP 55608 that a second dataflow may be included (e.g., the dataflow analyzer 55632 may provide the HCP 55608 with an opportunity to prevent the second dataflow from being used). As an illustrative example, a dataflow analyzer 55632 may determine that a robotic device (e.g., surgical element 55610) may not be able to effectively locate a probe tip. The dataflow analyzer 55632 may prompt an HCP 55608 (e.g., via a GUI) to activate a second dataflow associated with, for example, a cone beam CT that is energized and proximate to the patient (e.g., sensor 55625 and/or surgical element 55620 of operational environment 55600B) to locate the probe tip.

In examples, a second dataflow may not be available, and/or may not be enabled by the dataflow analyzer 55632 (e.g., as described with reference to FIGS. 6A, 6B, 7, 8, 9A, and/or 9B). In examples, a dataflow analyzer 55632 may determine that a robotic device (e.g., surgical element 55610) may not be able to effectively locate a probe tip. The dataflow analyzer 55632 may prompt an HCP 55608 (e.g., via a GUI), to connect a cone beam CT that is not in use (e.g., the dataflow analyzer 55632 may determine a relationship between the robotic device and the cone beam CT as described herein), and indicate to the HCP 55608 that a cone beam CT may be connected, positioned, energized and/or activated, to locate the probe tip.

In examples a dataflow analyzer 55632 may determine that a function of a surgical element 55610 may not achieve a desired outcome (e.g., then identify and add a second dataflow to achieve the desired outcome). As an illustrative example, a scope may traverse through a bronchi (e.g., via a robotic device). As the scope progresses further into the bronchial tree, scope position accuracy may decrease. Once position accuracy decreases past a threshold (e.g., a trigger condition is satisfied) a dataflow analyzer 55632 may determine to include additional position information (e.g., a second dataflow) to accurately determine the scope position (e.g., the dataflow analyzer 55632 may add position data from the CT to confirm the robotic device position to drive movement of the scope).

In examples, a dataflow analyzer 55632 may add a dataflow to optimize patient safety. In examples, a dataflow analyzer 55632 may indicate that an additional dataflow may be used to generate a specified output. As an illustrative example, a smoke evacuation device may be controlled by an energy activation device (e.g., output 55617) to clear a field of view for an HCP 55608 during a procedure. If an HCP 55608 is unable to see effectively (e.g., the smoke evacuation device is not effectively mitigating the issue) a scope is added as a dataflow to control the smoke evacuation rate. In examples, a dataflow from energy and/or a dataflow from an insufflation pressure may be used. In examples, the dataflow analyzer 55632 may determine if visual scope output, patient temperature, and/or another sensor may best maintain a field of view for the HCP 55608. In examples, ML model(s) 55636 may be trained to determine an optimized dataflow to be added.

In examples, a dataflow analyzer 55632 may determine that a second dataflow may be included based on ML model(s) 55636. As an illustrative example, a smoke evacuation device may be controlled by an energy activation device (e.g., output 55617) to clear a field of view for an HCP 55608 during a procedure. If an HCP 55608 is unable to see effectively (e.g., the smoke evacuation device is not effectively mitigating the issue) a scope occlusion is added as a dataflow to control the smoke evacuation rate. The dataflow analyzer may use ML model(s) 55636 to predict if/when the scope dataflow is needed (e.g., instead of waiting for an occlusion to limit the ability to function).

A dataflow analyzer 55632 may determine reduce the number of controlled variables (e.g., output 55617, 55627) in response to receiving an erroneous dataflow, and/or the like. In examples, a dataflow analyzer 55632 may limit the impact of an imbalance (e.g., an erroneous dataflow) by reducing the number of unknowns (e.g., reducing the control data associated with adjusting output characteristic(s) such as output 55617, 55627) without providing a second dataflow to create a closed loop (e.g., as indicated in bold boarders as part of operational environment 55600A, 55600B). In examples, one loop may remain closed, and/or a second loop may be allowed to remain open. In examples, a dataflow analyzer 55632 may determine that an erroneous dataflow is less reliable but related. Rather than using a second dataflow, the dataflow analyzer 55632 may use an internal dataflow (e.g., generated as part of system generated data, and/or the like). As an illustrative example, a visual camera may be a surgical element 55610, having a resolution (e.g., 480 * 620, and/or the like). The resolution may not be sufficient during a procedure. The dataflow analyzer 55632 may include a dataflow from an IR camera to increase the resolution. In examples, the IR camera and the visual camera images may be combined into an image.

In examples, a dataflow analyzer 55632 may utilize ML model(s) 55636 (e.g., a computer vision model). As an illustrative example, the dataflow analyzer 55632 may detect that endocutter connected, and prompt an HCP 55608 (e.g., a surgeon) to enable a perfusion detection model (e.g., the dataflow analyzer 55632 prompts the user to select the endocutter and/or the perfusion model).

As an illustrative example, as a scope is inserted into a bronchi, the EM position reliability may decrease. The dataflow analyzer 55632 may determine that additional position information is needed to ensure proper scope placement. The dataflow analyzer 55632 may add position data from a CT to confirm the position to drive movement of the scope. In examples, as the scope is inserted deeper within the body, the EM may lose accuracy (e.g., conflicts with reality). The dataflow analyzer 55632 may execute reverse kinematics to determine position. As an illustrative example a smoke evacuation may be driven by energy activation to prevent limitations of visibility. The HCP 55608 may be unable to see effectively (e.g., smoke evac is not effectively mitigating the issue). The HCP 55608 may request to change an input for the smoke evac to be dependent on scope occlusion instead of energy activation.

In examples, a dataflow analyzer 55632 may rebalance (e.g., determine a dataflow) by changing the effect of the dataflows. Changing the effect of dataflows may include a different gain factor (e.g., reducing a dataflow with a high level of noise). In examples, a dataflow analyzer 55632 may reduce the gain on a dataflow to improve reliability of the control data.

Historical data 55634 may store and/or include data associated with one or more past procedures. Historical data 55634 may be generated and/or received from surgical elements 55610, 55620, an HCP 55608 (e.g., via a GUI), and/or by the surgical computing system 55630. Historical data 55634 may be used as part of training data (e.g., to train ML model(s) 55636 as described herein). Historical data 55634 may be used by a surgical computing system 55630 to determine one or more relationships associated with surgical elements 55610, 55620). In examples, information associated with historical data 55634 may be included in a LUT to indicate one or more relationships between surgical elements 55610, 55620.

In examples, historical data 55634 may include control data, sensor data, operational data, system generated data, dataflow configuration information, and/or training data. Control data may include information associated with an output characteristic of a surgical element 55610, 55620. Sensor data may include data associated with a measurement of an environmental condition (e.g., one or more sensors) that may have been generated during a procedure. Operational data may include data associated with an HCP 55608 and/or patient 55641, such as historic procedural times, specific surgical elements used by an HCP during a procedure, historical records of a patient's physiological parameters, historical records of multiple patients' physiological parameters associated with a procedure, and/or the like. System generated data may include one or more inferred relationships (e.g., in a LUT), communication messages (e.g., interrogation message/response, configuration message/response, and/or the like), and/or the like, for one or more components of an operational environment. Training data, as described herein, may include data that is used to train an ML model(s) 55636 (e.g., to infer a relationship between components of an operational environment 55600A, 55600B).

In examples, historical data 55634 may include information associated with one or more dataflows. As an illustrative example, historical data 55634 may store information associated with identifying relationships between dataflows (e.g., based on configuration information), such as a surgical element ID, a communication protocol, scheduling and frequency information, a destination, security and access control credentials, a unit of measure, and/or the like.

A surgical computing system 55630 may include ML model(s) 55636. ML model(s) 55636 may receive, as an input, a dataflow, dataflow configuration information, control data, operational data, system generated data, historical data, and/or the like. In response to the received inputs, ML model(s) 55636 may determine one or more relationships associated with surgical elements 55610, 55620, determine whether a dataflow is erroneous, and/or determine an optimized dataflow that may be used to replace an erroneous dataflow and/or may be added to resolve an issue. The ML model(s) 55636 may transmit an output (e.g., a result, a determination, a recommendation, and/or the like) to dataflow analyzer 55632, based on one or more trained models. ML model(s) 55636 may generate individual training data items that form training data (e.g., to train one or more models). Training data items may include a procedure, historical data 55634 (e.g., control data, operational data, sensor data, dataflow configuration information, system generated data and/or the like), physiological parameters of a patient, one or more determined relationships, and/or the like.

ML model(s) 55636 may determine a relational link (e.g., a relationship) associated with a first surgical element 55610 and/or a second surgical element 55620. A relational link may be determined in real-time (e.g., during a procedure), or at another time (e.g., based on historical data 55634 as described herein). A relationship may be determined based on one or more aspects of a surgical element 55610, 55620 (e.g., dataflows, control data, sensor data, operational data, configuration information, and/or the like)as described herein. ML model(s) 55636 may store an indication that one or more aspects of surgical elements 55610, 55620 are related in, for example, historical data 55634 (e.g., via a LUT).

In examples, ML model(s) 55636 may determine that a relationship exists between sensor data associated with a second surgical element 55620 (e.g., sensor 55625) and sensor data associated with a first surgical element 55610 (e.g., sensor 55613). Sensor data may be related if, for example, sensor data shares a similar unit of measure, the sensor data measure a similar physiological parameter of a patient's body, the sensors are proximate to one another during a procedure, and/or the like.

In examples, ML model(s) 55363 may determine that a relationship exists between sensor data associated with a second surgical element 55620 (e.g., sensor 55625) and/or control data generated for a first surgical element 55610 (e.g., control data used to control output 55617). Sensor data associated with a second surgical element 55620 (e.g., sensor 55625) may be related to control data generated for a first surgical element 55610 if, for example, a sensor 55625 measures an output characteristic of the first surgical element 55610 as described herein.

In examples, ML model(s) 55636 may determine that a relationship exists based on dataflow configuration information (e.g., based on a surgical element ID, a communication protocol, scheduling and frequency information, a destination, security and access control credentials, and/or a unit of measure), for a surgical elements 55610, 55620. As an illustrative example, a dataflow analyzer 55632 may transmit to ML model(s) 55636, a first dataflow from a laparoscopic tool (e.g., a dataflow indicating a measured temperature of an insufflated cavity via sensor 55625) and a second dataflow from a heating pad (e.g., a dataflow indicating a measured temperature near an insufflated cavity via sensor 55613 and/or a power level associated with heat generated by the heating pad (e.g., via output 55617)). ML model(s) 55636 may determine that an increase in temperature, as measured by the laparoscopic tool, is caused by the power output associated with the heating pad. ML model(s) may indicate, to dataflow analyzer 55632, that the temperature sensor associated with the laparoscopic tool may be used to control the power output of the heating pad.

ML model(s) 55636 may determine whether a dataflow is erroneous. As described herein, an erroneous dataflow may be caused by, for example, an incorrectly calibrated sensor, a misplaced and/or improperly installed sensor (e.g., interference by a heat source), calibration drift, contamination, electromagnetic interference, a failed sensor, operator error, misalignment (e.g., in the case of a position sensor), and/or the like. ML model(s) 55636 be trained to detect (e.g., determine) that a dataflow erroneous in real-time (e.g., based on operational data, sensor, data, system generated data, and/or control data). In examples, ML model(s) 55636 may determine that a dataflow is erroneous based on a comparison of control data, sensor data, operational data, and/or system generated data to historical data. As an illustrative example, ML model(s) 55636 may determine that a dataflow (e.g., associated with a temperature indication) is erroneous if a first temperature sensor increases while one or more related temperature sensors remain at a constant temperature.

ML model(s) 55636 may determine an optimized dataflow that may be used to replace an erroneous dataflow. ML model(s) 55636 may determine that a dataflow is an optimized dataflow based on dataflow configuration information, a procedure, one or more physiological parameters of a patient, historical data, one or more determined relationships, an input from an HCP 55608, and/or the like. An optimized dataflow may be selected from a plurality of dataflows, to provide sensor data to a dataflow analyzer 55632 that most closely resembles and/or represents sensor data previously sent in an erroneous dataflow.

For example, ML model(s) 55636 may determine that a dataflow (e.g., including sensor data from sensor 55625) may provide stable, reliable, information that may be used by the dataflow analyzer 55632 to generate reliable control data. As an illustrative example, an optimized dataflow may be different (e.g., sensor data from a first temperature sensor may be more accurate than a second temperature sensor) for a patient that is susceptible to hypothermia (e.g., an elderly patient, a pediatric patient, and/or the like) versus a patient that may be more resistant to hypothermia (e.g., a patient with a high body-mass index, patients with high basal metabolic rates, and/or the like).

ML model(s) 55636 may estimate sensor data associated with a failed sensor 55613 during a procedure. In examples, ML model 55636 may receive control data, sensor data, operational data, and/or system generated data, and estimate an environmental condition of an operational environment 55600A, 55600B (e.g., during a procedure) based on previous measurements during similar portions of a procedure.

### Example Routine for Re-Balancing the Number of Unknowns and Dataflows

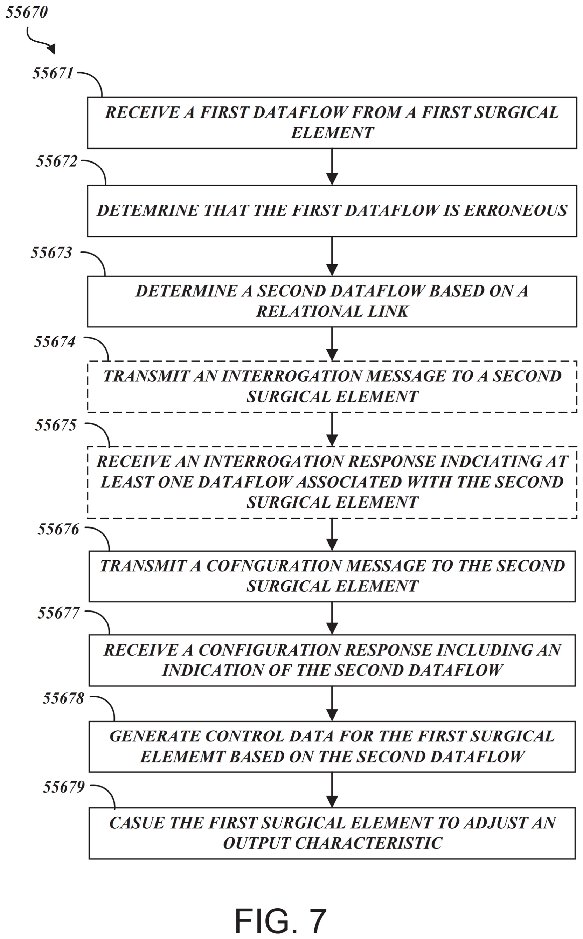

FIG. 7 illustrates an example routine 55670 for re-balancing the number of unknowns and dataflows (e.g., data streams). Example routine 55670 may be executed by, for example, surgical computing system 55630 (e.g., dataflow analyzer 55632) of operational environment 55600A, 55600B. The example routine 55670 begins at block 55671.

At block 55671, a dataflow analyzer 55632 may receive a first dataflow from a first surgical element 55610. The dataflow may include sensor data, operational data, system generated data, control data, dataflow configuration information, and/or the like. In examples, dataflow analyzer 55632 may receive sensor data from sensor 55613. The sensor data may be a signal indicating a measurement of an environmental condition during a procedure (e.g., a current, voltage, a pressure, a force applied, a distance, a vibration, an orientation, a flow rate, a status, and/or the like).

At block 55672, a dataflow analyzer 55632 may determine that the first dataflow is erroneous based on a determination that control data exceeds a threshold, a determination that the first dataflow is unavailable, a determination that the physiological parameter of the patient exceeds a patient safety threshold, based on a comparison of sensor data to historical data (e.g., past sensor data and/or another data type), and/or based on an output of ML model(s) 55636.

At block 55673, a dataflow analyzer 55632 may determine a second dataflow based on a relational link (e.g., a relationship between surgical elements). A relationship may be defined in a LUT, determined by an HCP 55608, and/or determined by ML model(s) 55636 as described herein. As an illustrative example, a dataflow analyzer 55632 may transmit, to ML model(s) 55636, a first dataflow from a laparoscopic tool (e.g., a dataflow indicating a measured temperature of an insufflated cavity via sensor 55625) and a second dataflow from a heating pad (e.g., a dataflow indicating a measured temperature near an insufflated cavity via sensor 55613 and/or a power level associated with heat generated by the heating pad (e.g., via output 55617)). The ML model(s) 55636 may determine that an increase in temperature, as measured by the laparoscopic tool, is caused by the power output associated with the heating pad. ML model(s) may indicate a relationship between the laparoscopic tool and the heating pad to the dataflow analyzer 55632 (e.g., that the temperature sensor associated with the laparoscopic tool may be used to control the power output of the heating pad).

Optionally at block 55674, a dataflow analyzer 55632 may transmit an interrogation message to a second surgical element 55620. As described herein, an interrogation message may request, among other things, confirmation that a second surgical element 55620 includes a compatible and/or available dataflow (e.g., based on a determined relationship).

Optionally, at block 55675, a dataflow analyzer 55632 may receive an interrogation response indicating at least one dataflow associated with the second surgical element 55620. In examples, the dataflow analyzer 55632 may receive an interrogation response indicating that a second dataflow is not available. In examples, an interrogation response may indicate an update to information associated surgical element 55610, 55620, and/or an acknowledgement of the information associated with the surgical element 55610, 55620. An update to one or more determined relationships may include an indication of a second sensor that may be related to a failed sensor (e.g., sensor 55625 may be related to sensor 55613 in operational environment 55600B). In examples, the interrogation response may indicate that a surgical element 55620 may be configured to transmit the requested dataflow (e.g., that a dataflow is available, that one or more dataflows may be removed (e.g., taken off-line) to send the requested dataflow, and/or the like).

At block 55676, a dataflow analyzer 55632 may transmit a configuration message to the second surgical element 55620. A configuration message may be sent in response to a received interrogation response, based on a user input (e.g., an HCP 55608 via a GUI), and/or as determined by the dataflow analyzer 55632 (e.g., based on a determination that a dataflow is sending erroneous data and/or the like). A configuration message may include a request for sensor data (e.g., a dataflow) based on one or more relationships (e.g., as determined by the dataflow analyzer 55632, ML model(s) 55636). A configuration message may include dataflow configuration information as described herein, to configure a surgical element 55620 to transmit a second dataflow.

At block 55677, a dataflow analyzer 55632 may receive a configuration response including an indication of the second dataflow. As described herein, a configuration response may include dataflow configuration information and/or a dataflow associated with a surgical element 55620. Dataflow configuration information may include a surgical element ID, an indication whether a dataflow is available, an update and/or acknowledgement to dataflow configuration information, a unit of measure, a communication protocol (RS-232, Ethernet, TCP/IP, Bluetooth, and/or the like), scheduling and/or frequency information (e.g., a time and/or frequency that sensor data is to be sent), a destination (e.g., port information associated with the surgical element controller 556611), and/or security and/or access control credentials.

At block 55678, a dataflow analyzer 55632 may generate control data for the first surgical element based on the second dataflow (e.g., from the second surgical element). Additionally and/or optionally, a dataflow analyzer 55632 may generate control data based on a user input (e.g., by an HCP 55608 via a GUI) and/or as determined by the dataflow analyzer 55632. As described herein, control data may include information associated with, for example, a control variable setpoint (e.g., associated with a motor speed, a temperature, a flow rate, and/or the like), a current and/or voltage (e.g., associated with an electrosurgical tool, and/or the like), an instruction (e.g., to generate an image from a camera and/or the like), and/or a power level (e.g., of a heating pad, a laser ablation tool, and/or the like).

At block 55679, a dataflow analyzer 55632 may cause the first surgical element to adjust an output characteristic (e.g., based on the transmitted control data). Advantageously, a dataflow analyzer 55632 may send control data to a first surgical element (e.g., that may have a failed sensor), based on a second dataflow to avoid pausing, cancelling, and/or continuing a procedure with a reduced number of surgical elements.

### Example Communication Diagram for Re-Balancing the number of Unknowns and Dataflows

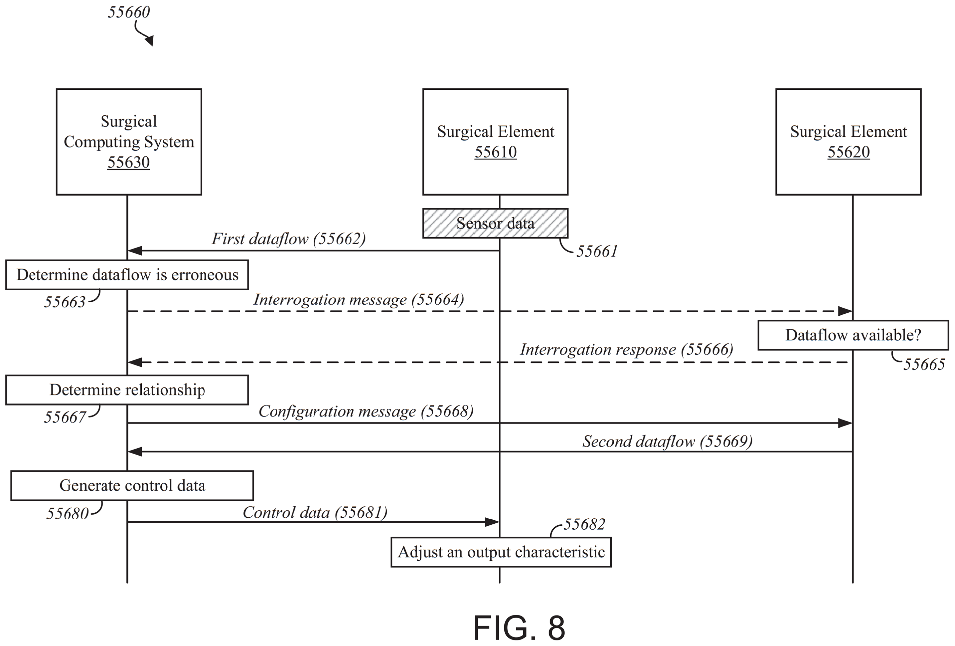

FIG. 8 illustrates an example communication diagram 55660 for re-balancing the number of unknowns and dataflows (e.g., data streams). The example communication diagram 55660 may be described with reference to one or more components of operational environment 55600B, including a surgical computing system 55630, a surgical element 55610, and/or a surgical element 55620. Advantageously, a surgical computing system 55630 (e.g., dataflow analyzer 55632) may use sensor data (e.g., a second dataflow) from a second surgical element to generate control data for the first surgical element (e.g., instead of pausing a procedure to replace the failed surgical element, cancelling a procedure, and/or continuing a procedure with a reduced number of surgical elements). The surgical computing system 55630 may avoid pausing, canceling, and/or compromising safety during a procedure by determining a second dataflow based on one or more associated relationships between surgical elements.

In examples, surgical element 55610 may generate sensor data at 55661. The sensor data may be erroneous (e.g., as indicated in shading). As described herein, erroneous data be caused by an incorrectly calibrated sensor, a misplaced and/or improperly installed sensor (e.g., interference by a heat source), calibration drift, contamination, electromagnetic interference, a failed sensor, operator error, misalignment (e.g., in the case of a position sensor), and/or the like.

Erroneous sensor data at 55661 may be transmitted from surgical element 55610 to surgical computing system 55630 via a first dataflow 55662. A first dataflow may include sensor data, operational data, system generated data, control data, dataflow configuration information, and/or the like. Although FIG. 8 depicts erroneous sensor data at 55661, surgical element 55610 may transmit a dataflow 55662 including erroneous operational data, system generated data, control data, and/or the like.

The surgical computing system 55630 may receive the first dataflow 55662 including erroneous sensor data 55661. The surgical computing system 55630 may determine that a dataflow is erroneous at 55663 based on a determination that control data exceeds a threshold, a determination that the first dataflow is unavailable, a determination that the physiological parameter of the patient exceeds a patient safety threshold, based on a comparison of sensor data to historical data (e.g., past sensor data and/or another data type), and/or based on an output of ML model(s) 55636.

The surgical computing system 55630 may transmit an interrogation message 55664 to surgical element 55620. An interrogation message may be transmitted in response to determining that a dataflow is erroneous. Additionally and/or optionally, an interrogation message may be transmitted in response to a user input (e.g., an HCP via a GUI), and/or as determined by the surgical computing system 55630. As described herein, an interrogation message may request information associated with one or more dataflows of a surgical element 55620. The interrogation message may include dataflow configuration information associated with a surgical element 55610, 55620 (e.g., to determine whether a second dataflow is available).

A surgical element 55620 may receive an interrogation message 55664 and determine if a dataflow is available at 55665. In examples, a surgical element 55620 may determine that a second dataflow is available if a first dataflow is removed (e.g., taken off-line and/or replaced by the second dataflow on a configured channel). In examples, a second surgical element may determine that a second dataflow is available via a second channel and/or determine that a second dataflow may share a channel with a first dataflow.

An interrogation response 55666 may be sent from the surgical element 55620 to the surgical computing system 55630. The interrogation response 55666 may be sent in response to determining whether a dataflow is available at 55665. As described herein, an interrogation response may include an indication whether a second dataflow is available, an indication of an update to information associated with a surgical element 55620, and/or an acknowledgement of the information associated with surgical element 55610, 55620 (e.g., among other dataflow configuration information).

A surgical computing system 55630 may determine a relationship at 55667. In examples, a relationship may be determined based on the received interrogation response 55666. As described herein, a relationship (e.g., between surgical element 55610 and/or surgical element 55620) may be determine based on one or more aspects of a surgical element 55610, 55620 (e.g., dataflows, control data, sensor data, operational data, configuration information, and/or the like), based on data associated with a LUT, and/or based on an output of ML model(s) 55636.

If the surgical computing system 55630 determines that a relationship exists, the surgical computing system 55630 may transmit a configuration message 55668. As described herein, a configuration message may include information associated with configuring a surgical element 55610, 55620. As an illustrative example, dataflow analyzer 55632 may send a configuration message to surgical element 55620, to configure a second dataflow (e.g., via 55622 and/or if determined by the dataflow analyzer via 55629) for receiving sensor data associated with sensor 55625, based on a determination that sensor 55625 is related to failed sensor 55613.

Surgical element 55620 may receive configuration message 55668, and in response, configure the requested dataflow (e.g., select, generate and/or transmit the sensor data requested in the configuration message 55668). In response to receiving the configuration message 55668, surgical element 55620 may send a second dataflow 55682 to the surgical computing system 55630. The second dataflow 55669 may include, among other things, sensor data based on one or more relationships as determined at 55667.

Surgical computing system 55630 may receive the second dataflow 55669, and in response, generate control data at 55680. As described herein, control data may include information associated with, for example, a control variable setpoint, a current and/or voltage, an instruction, and/or a power level. As an illustrative example, control data may include a setpoint for the speed of a cutting tool, a voltage and/or current setpoint of an electrosurgical tool, a flow rate for an infusion pump, a position of a robotic arm, and/or the like.

The surgical computing system 55630 may transmit control data 55681 to a surgical element 55610, to cause the surgical element 55610 to adjust an output characteristic associated with output 55617. The output may adjust an output characteristic at 55682 based on the information associated with a control variable setpoint, a current and/or voltage, an instruction, a power level, and/or the like, as indicated in the control data.

As described herein, the control data 55681 may be generated based on a sensor of a second surgical element (e.g., surgical element 55620), and transmitted to a first surgical element (e.g., surgical element 55610). The control data may be used, for example, to rebalance the number of unknowns and the number of data streams (e.g., dataflows). Surgical element 55610 may receive the control data 55681 and adjust an output characteristic at 55682 as described herein.

### Example Aspects for Re-Balancing Unknowns and Dataflows

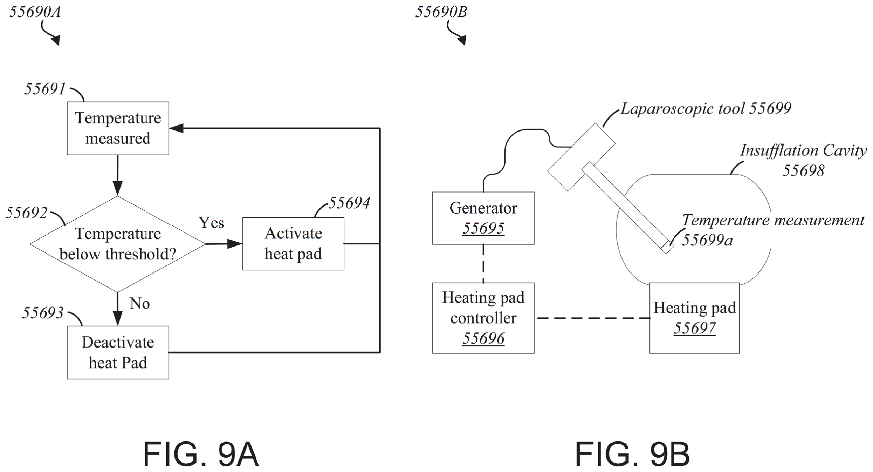

FIGS. 9A and 9B illustrate an example implementation of rebalancing unknowns and dataflows (e.g., data streams) via an insufflated temperature regulation system. Advantageously, routine 55690A and/or environment 55690B may immediately and/or continuously estimate the insufflation cavity temperature, improve heating pad performance and efficiency, and/or minimize patient temperature fluctuations throughout surgery. The following may be described based on a procedure associated with an elderly, low BMI patient undergoing a necessary surgery that is having complications. Temperature measurement 55699a of the laparoscopic tool 55699 may measure the insufflated air temperature of insufflated cavity 55698, and/or proactively the generator 55695 may determine that the patient is at risk of hypothermia without intervention. A warning may be presented (e.g., to an HCP via a GUI) to increase a heat pad setting (e.g., heating pad 55697) or a signal (e.g., control data) may be sent to the heating pad controller 55696, to increase temperature.

A patient may avoid further complication from an already high risk procedure based on the routine 55960A and/or environment 55690B as described herein. In examples, lengthy insufflated surgical procedures may have a risk of causing hypothermia despite the presence of heating pads. During long surgical procedures which may have leaky ports, frequent smoke evacuations, patient response to generate anesthesia, and high gas flow rates, the internal body temperature of the patient may drop to unsafe levels.

To address this issue, supplemental heating in the form of heating pads 55697 may be provided to the patient (e.g., insufflation cavity 55698). To automatically match the patient specific heating needs, a laparoscopic tool 55699 may detect the ambient temperature of the insufflated cavity (e.g., via temperature measurement 55699a). The temperature measurement 55699a may regulate the amount of heating provided to the patient. Heating pads 55697 may be further improved by the temperature measurement 55699a from the energy tool (e.g., heating pad controller 55696). The provided temperature may be used to autoregulate the heat setting of a patient heating pad 55697 to minimize temperature fluctuations throughout a surgery (e.g., autoregulated by the generator 55695).

FIG. 9A illustrates an example routine 55690A for temperature control. FIG. 9B illustrates an example environment 55690B for executing one or more functions associated with routine 55690A. The example environment 55690B may include a generator 55695 (e.g., similar to surgical computing system 55630 of FIG. 6B) , a heating pad controller 55696 (e.g., similar to surgical element controller 55611 of FIG. 6B), a heating pad 55697 (e.g., similar to output 55617 of FIG. 6B), an insufflated cavity 55698 (e.g., similar to a patient 55641 of FIG. 6B), and/or a temperature measurement 55699a (e.g., similar to sensor 55625 of FIG. 5B).

Routine 55690A begins at block 55961 where a temperature is measured by temperature measurement 55699a of a laparoscopic tool 55699. The temperature may be measured for an insufflated cavity 55698. In examples, temperature measurement 55699a may include one or more similar features and/or functions as sensor 55625 of operational environment 55600B.

At decision node 55692, a generator 55695 (e.g., a dataflow analyzer 55632) may determine whether the temperature measurement 55699a is below a threshold. A temperature estimator model (e.g., as part of generator 55695) may determine the environmental temperature throughout a procedure. If the insufflation cavity 55698 temperature is lower than a threshold (e.g., an internal body temperature of the patient), the routine may continue to block 55694. If the insufflation cavity 55698 temperature is not below a threshold (e.g., above an internal body temperature of the patient) the routine may continue to block 55963.

At block 55693, generator 55695 may transmit a message (e.g., as part of control data) indicating that heating pad controller 55696 may deactivate heating pad 55697. After heating pad 55697 is deactivated, the routine 55690A may loop back to block 55691 and/or end.

At block 55964, generator 55695 may transmit a message (e.g., as part of control data) indicating that heating pad controller 55696 may activate heating pad 55697. After heating pad 55697 is activated, the routine 55690A may loop back to block 55691 and/or end.

The following is a non-exhaustive list of examples that may or may not be claimed:
1. A device, comprising:
   a processor configured to:
   receive a first dataflow from a first surgical element, wherein the first dataflow is associated with a physiological parameter of a patient;
   determine based on the first dataflow, that the physiological parameter of the patient exceeds a patient safety threshold;
   determine a second dataflow associated with a second surgical element, wherein the determination is based on an indication of a relational link associated with control data for the second surgical element, the first dataflow, and the physiological parameter of the patient;
   transmit, to the second surgical element, a configuration message comprising an indication that the physiological parameter of the patient exceeded the patient safety threshold, and a request to configure the second dataflow to receive control data associated with the first surgical element;
   generate the control data associated with the first surgical element, wherein the control data indicates an adjustment to an output characteristic associated with the second surgical element;
   transmit, to the second surgical element, a control message, wherein the control message comprises an indication of the control data associated with the first surgical element; and
   cause the output characteristic associated with the second surgical element to be adjusted based on the control data associated with the first surgical element.
2. The device of example 1 wherein the physiological parameter of the patient satisfies a threshold if a heart rate exceeds an operational window of 40-120 beats per minute, an oxygen saturation of the patient decreases below 90%, or a core body temperature of the patient is less than 89 degrees Fahrenheit.
3. The device of any of the preceding examples, wherein the configuration message further comprises a surgical element ID, a communication protocol, and security or access control credentials.
4. The device of any of the preceding examples, wherein the relational link is determined based on a lookup table (LUT), wherein the LUT indicates a relationship between the control data for the second surgical element, the first dataflow, and the physiological parameter of the patient.
5. The device of any one of examples 1-3, wherein the relational link is determined based on a machine learning (ML) model, wherein the ML model is trained based on a training data set, wherein the training data set comprises a plurality of dataflows and a plurality of control data associated with the physiological parameter of the patient.
6. The device of any of the preceding examples, wherein the processor is further configured to:
   based on the first dataflow, determine that the physiological parameter of the patient satisfies a second threshold, wherein the second threshold is associated with an acceptable operational range; and
   transmit a second configuration message, wherein the second configuration message comprises an indication to reconfigure the second dataflow of the second surgical element to remove the control data associated with the first surgical element.
7. The device of any of the preceding examples, wherein the processor is further configured to:
   in response to the determination that the physiological parameter of the patient exceeded the patient safety threshold, transmit an interrogation message to the second surgical element, wherein the interrogation message requests dataflow configuration information for the second surgical element and an indication of control data for the second surgical element that is associated with the physiological parameter of the patient; and
   receive an interrogation response indicating at least one physiological parameter of the patient associated with the second surgical element.
8. The device of example 7, wherein the dataflow configuration information comprises at least one of a communication protocol, a frequency, a destination, or access control credentials.
9. A method comprising:
   receiving a first dataflow from a first surgical element, wherein the first dataflow is associated with a physiological parameter of a patient;
   determining based on the first dataflow, that the physiological parameter of the patient exceeds a patient safety threshold;
   determining a second dataflow associated with a second surgical element, wherein the determination is based on an indication of a relational link associated with control data for the second surgical element, the first dataflow, and the physiological parameter of the patient;
   transmitting, to the second surgical element, a configuration message comprising an indication that the physiological parameter of the patient exceeded the patient safety threshold, and a request to configure the second dataflow to receive control data associated with the first surgical element;
   generating the control data associated with the first surgical element, wherein the control data indicates an adjustment to an output characteristic associated with the second surgical element;
   transmitting, to the second surgical element, a control message, wherein the control message comprises an indication of the control data associated with the first surgical element; and
   causing the output characteristic associated with the second surgical element to be adjusted based on the control data associated with the first surgical element.
10. The method of example 9, wherein the physiological parameter of the patient satisfies a threshold if a heart rate exceeds an operational window of 40-120 beats per minute, an oxygen saturation of the patient decreases below 90%, or a core body temperature of the patient is less than 89 degrees Fahrenheit.
11. The method of any of the preceding examples, wherein the configuration message further comprises a surgical element ID, a communication protocol, and security or access control credentials.
12. The method of any of the preceding examples, wherein the relational link is determined based on a lookup table (LUT), wherein the LUT indicates a relationship between the control data for the second surgical element, the first dataflow, and the physiological parameter of the patient.
13. The method of any one of examples 9-12, wherein the relational link is determined based on a machine learning (ML) model, wherein the ML model is trained based on a training data set, wherein the training data set comprises a plurality of dataflows and a plurality of control data associated with the physiological parameter of the patient.
14. The method of any of the preceding examples, wherein the method further comprises:
   based on the first dataflow, determining that the physiological parameter of the patient satisfies a second threshold, wherein the second threshold is associated with an acceptable operational range; and
   transmitting a second configuration message, wherein the second configuration message comprises an indication to reconfigure the second dataflow of the second surgical element to remove the control data associated with the first surgical element.
15. The method of any of the preceding examples, wherein the method further comprises:
   in response to the determination that the physiological parameter of the patient exceeded the patient safety threshold, transmitting an interrogation message to the second surgical element, wherein the interrogation message requests dataflow configuration information for the second surgical element and an indication of control data for the second surgical element that is associated with the physiological parameter of the patient, wherein the dataflow configuration information comprises at least one of a communication protocol, a frequency, a destination, or access control credentials; and
   receiving an interrogation response indicating at least one physiological parameter of the patient associated with the second surgical element.
16. A device comprising:
   a processor configured to:
   determine based on a first dataflow, that a physiological parameter of a patient exceeds a patient safety threshold;
   determine a second dataflow associated with a second surgical element, wherein the determination is based on an indication of a relational link associated a first surgical element and the second surgical element;
   generate control data associated with the first surgical element, wherein the control data indicates an adjustment to an output characteristic associated with the second surgical element;
   transmit, to the second surgical element a control message, wherein the control message comprises an indication of the control data associated with the first surgical element; and
   cause the output characteristic associated with the second surgical element to be adjusted based on the control data associated with the first surgical element.
17. The device of example 16, wherein the relational link is determined based on a lookup table (LUT), wherein the LUT indicates a relationship between the control data for the second surgical element, the first dataflow, and the physiological parameter of the patient.
18. The device of example 16, wherein the relational link is determined based on a machine learning (ML) model, wherein the ML model is trained based on a training data set, wherein the training data set comprises a plurality of dataflows and a plurality of control data associated with the physiological parameter of the patient.
19. The device of any of the preceding examples, wherein the processor is further configured to:
   based on the first dataflow, determine that the physiological parameter of the patient satisfies a second threshold; and
   transmit a second configuration message, wherein the second configuration message comprises an indication to reconfigure the second dataflow of the second surgical element to remove the control data associated with the first surgical element.
20. The device of any of the preceding examples, wherein the processor is further configured to:
   in response to the determination that the physiological parameter of the patient exceeded the patient safety threshold, transmit an interrogation message to the second surgical element, wherein the interrogation message requests control data for the second surgical element that is associated with the physiological parameter of the patient; and
   receive an interrogation response indicating whether the second surgical element has control data associated with the physiological parameter of the patient.

Although features and elements are described above in particular combinations, one of ordinary skill in the art will appreciate that each feature or element can be used alone or in any combination with the other features and elements. In addition, the methods described herein may be implemented in a computer program, software, or firmware incorporated in a computer-readable medium for execution by a computer or processor. Examples of computer-readable media include electronic signals (transmitted over wired or wireless connections) and computer-readable storage media. Examples of computer-readable storage media include, but are not limited to, a read only memory (ROM), a random access memory (RAM), a register, cache memory, semiconductor memory devices, magnetic media such as internal hard disks and removable disks, magnetooptical media, and optical media such as CD-ROM disks, and digital versatile disks (DVDs). A processor in association with software may be used to implement a radio frequency transceiver for use in a WTRU, UE, terminal, base station, RNC, or any host computer.

## Claims

1. A device comprising a processor configured to:
receive a first dataflow from a first surgical element, wherein the first dataflow is associated with a physiological parameter of a patient;
determine based on the first dataflow, that the physiological parameter of the patient exceeds a patient safety threshold;
determine a second dataflow associated with a second surgical element, wherein the determination is based on an indication of a relational link associated with control data for the second surgical element, the first dataflow, and the physiological parameter of the patient;
transmit, to the second surgical element, a configuration message comprising an indication that the physiological parameter of the patient exceeded the patient safety threshold, and a request to configure the second dataflow to receive control data associated with the first surgical element;
generate the control data associated with the first surgical element, wherein the control data indicates an adjustment to an output characteristic associated with the second surgical element;
transmit, to the second surgical element, a control message, wherein the control message comprises an indication of the control data associated with the first surgical element; and
cause the output characteristic associated with the second surgical element to be adjusted based on the control data associated with the first surgical element.

2. The device of claim 1, wherein the processor is further configured to:
based on the first dataflow, determine that the physiological parameter of the patient satisfies a second threshold, wherein the second threshold is associated with an acceptable operational range; and
transmit a second configuration message, wherein the second configuration message comprises an indication to reconfigure the second dataflow of the second surgical element to remove the control data associated with the first surgical element.

3. The device of claim 1 or claim 2, wherein the processor is further configured to:
in response to the determination that the physiological parameter of the patient exceeded the patient safety threshold, transmit an interrogation message to the second surgical element, wherein the interrogation message requests dataflow configuration information for the second surgical element and an indication of control data for the second surgical element that is associated with the physiological parameter of the patient; and
receive an interrogation response indicating at least one physiological parameter of the patient associated with the second surgical element,
optionally wherein the dataflow configuration information comprises at least one of a communication protocol, a frequency, a destination, or access control credentials.

4. A method performed by a processor, the method comprising:
receiving a first dataflow from a first surgical element, wherein the first dataflow is associated with a physiological parameter of a patient;
determining based on the first dataflow, that the physiological parameter of the patient exceeds a patient safety threshold;
determining a second dataflow associated with a second surgical element, wherein the determination is based on an indication of a relational link associated with control data for the second surgical element, the first dataflow, and the physiological parameter of the patient;
transmitting, to the second surgical element, a configuration message comprising an indication that the physiological parameter of the patient exceeded the patient safety threshold, and a request to configure the second dataflow to receive control data associated with the first surgical element;
generating the control data associated with the first surgical element, wherein the control data indicates an adjustment to an output characteristic associated with the second surgical element;
transmitting, to the second surgical element, a control message, wherein the control message comprises an indication of the control data associated with the first surgical element; and
causing the output characteristic associated with the second surgical element to be adjusted based on the control data associated with the first surgical element.

5. The device of any one of claims 1 to 3 or the method of claim 4, wherein the physiological parameter of the patient satisfies a threshold if a heart rate exceeds an operational window of 40-120 beats per minute, an oxygen saturation of the patient decreases below 90%, or a core body temperature of the patient is less than 89 degrees Fahrenheit.

6. The device of any one of claims 1 to 3 or the method of claim 4 or claim 5, wherein the configuration message further comprises a surgical element ID, a communication protocol, and security or access control credentials.

7. The method of any one of claims 4 to 6, wherein the method further comprises:
based on the first dataflow, determining that the physiological parameter of the patient satisfies a second threshold, wherein the second threshold is associated with an acceptable operational range; and
transmitting a second configuration message, wherein the second configuration message comprises an indication to reconfigure the second dataflow of the second surgical element to remove the control data associated with the first surgical element.

8. The method of any one of claims 4 to 7, wherein the method further comprises:
in response to the determination that the physiological parameter of the patient exceeded the patient safety threshold, transmitting an interrogation message to the second surgical element, wherein the interrogation message requests dataflow configuration information for the second surgical element and an indication of control data for the second surgical element that is associated with the physiological parameter of the patient, wherein the dataflow configuration information comprises at least one of a communication protocol, a frequency, a destination, or access control credentials; and
receiving an interrogation response indicating at least one physiological parameter of the patient associated with the second surgical element.

9. A device comprising a processor configured to:
determine based on a first dataflow, that a physiological parameter of a patient exceeds a patient safety threshold;
determine a second dataflow associated with a second surgical element, wherein the determination is based on an indication of a relational link associated a first surgical element and the second surgical element;
generate control data associated with the first surgical element, wherein the control data indicates an adjustment to an output characteristic associated with the second surgical element;
transmit, to the second surgical element a control message, wherein the control message comprises an indication of the control data associated with the first surgical element; and
cause the output characteristic associated with the second surgical element to be adjusted based on the control data associated with the first surgical element.

10. The device of any one of claims 1 to 3 and 9 or the method of any one of claims 4 to 8, wherein the relational link is determined based on a lookup table, LUT, wherein the LUT indicates a relationship between the control data for the second surgical element, the first dataflow, and the physiological parameter of the patient.

11. The device of any one of claims 1 to 3, 9, and 10 or the method of any one of claims 4 to 8, wherein the relational link is determined based on a machine learning, ML, model, wherein the ML model is trained based on a training data set, wherein the training data set comprises a plurality of dataflows and a plurality of control data associated with the physiological parameter of the patient.

12. The device of any one of claims 9 to 11, wherein the processor is further configured to:
based on the first dataflow, determine that the physiological parameter of the patient satisfies a second threshold; and
transmit a second configuration message, wherein the second configuration message comprises an indication to reconfigure the second dataflow of the second surgical element to remove the control data associated with the first surgical element.

13. The device of any one of claims 9 to 12, wherein the processor is further configured to:
in response to the determination that the physiological parameter of the patient exceeded the patient safety threshold, transmit an interrogation message to the second surgical element, wherein the interrogation message requests control data for the second surgical element that is associated with the physiological parameter of the patient; and
receive an interrogation response indicating whether the second surgical element has control data associated with the physiological parameter of the patient.

14. A computer-readable medium comprising instructions which, when executed by a processor, cause the processor to carry out the method of any one of claims 4 to 8, 10, and 11.
